(19) Europäisches Patentamt
European Patent Office
Office européen des brevets

(11) **EP 4 118 653 B1**

(12) **EUROPEAN PATENT SPECIFICATION**

(45) Date of publication and mention
of the grant of the patent:
**17.07.2024 Bulletin 2024/29**

(21) Application number: **21715455.8**

(22) Date of filing: **11.03.2021**

(51) International Patent Classification (IPC):
*G16B 20/20* (2019.01)

(52) Cooperative Patent Classification (CPC):
**G16B 20/20;** G16B 40/00

(86) International application number:
**PCT/US2021/021994**

(87) International publication number:
**WO 2021/183821 (16.09.2021 Gazette 2021/37)**

(54) **METHODS FOR CLASSIFYING GENETIC MUTATIONS DETECTED IN CELL-FREE NUCLEIC ACIDS AS TUMOR OR NON-TUMOR ORIGIN**

VERFAHREN ZUR KLASSIFIZIERUNG VON IN ZELLFREIEN NUKLEINSÄUREN ERKANNTEN GENETISCHEN MUTATIONEN ALS TUMOR- ODER NICHTTUMORURSPRUNG

PROCÉDÉS DE CLASSIFICATION DE MUTATIONS GÉNÉTIQUES DÉTECTÉES DANS DES ACIDES NUCLÉIQUES ACELLULAIRES EN TANT QU'ORIGINE TUMORALE OU NON TUMORALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.03.2020 US 202062988306 P**

(43) Date of publication of application:
**18.01.2023 Bulletin 2023/03**

(73) Proprietor: **Guardant Health, Inc.**
**Palo Alto, CA 94304 (US)**

(72) Inventors:
• **YEN, Jennifer**
**Redwood City, California 94063 (US)**
• **CHURSOV, Andrey**
**Redwood City, California 94063 (US)**

(74) Representative: **Carpmaels & Ransford LLP**
**One Southampton Row**
**London WC1B 5HA (GB)**

(56) References cited:
**WO-A1-2019/169042      WO-A1-2019/236478**

• **RAZAVI PEDRAM ET AL: "High-intensity sequencing reveals the sources of plasma circulating cell-free DNA variants", NATURE MEDICINE, NATURE PUB. CO, NEW YORK, vol. 25, no. 12, 25 November 2019 (2019-11-25), pages 1928 - 1937, XP036953312, ISSN: 1078-8956, [retrieved on 20191125], DOI: 10.1038/S41591-019-0652-7**

• **SUKHAI MAHADEO A. ET AL: "Somatic Tumor Variant Filtration Strategies to Optimize Tumor-Only Molecular Profiling Using Targeted Next-Generation Sequencing Panels", THE JOURNAL OF MOLECULAR DIAGNOSTICS, vol. 21, no. 2, 1 March 2019 (2019-03-01), pages 261 - 273, XP055827736, ISSN: 1525-1578, Retrieved from the Internet <URL:https://www.jmdjournal.org/article/S1525-1578(17)30598-6/pdf> DOI: 10.1016/j.jmoldx.2018.09.008**

EP 4 118 653 B1

**Description**

**BACKGROUND**

Liquid biopsy tests can be used to profile circulating tumor nucleic acids in blood samples from patients for the purpose of, for example, detecting cancer at an early stages, selecting therapy, and monitoring disease progression and/or minimal residual disease. Circulating plasma cell-free tumor DNA (ctDNA) are small DNA fragments from apoptotic and necrotic tumor cells or from circulating tumor cells (CTCs) that have been introduced into the bloodstream. ctDNA is only the portion of cell-free DNA (cfDNA) specifically released from cancer cells, while most of the cfDNA in a given sample typically originates from normal non-cancerous cells, including from normal leukocytes, hematopoietic stem cells (HSCs), or other early blood cell progenitors that undergo apoptosis or necrosis during clonal hematopoietic processes. One problem associated with many liquid biopsy tests is differentiating ctDNA from other cfDNA in patient samples.

Accordingly, there remains a need for methods and related aspects to differentiate tumor and non-tumor origin nucleic acid variants detected in cell-free nucleic acid (cfNA) samples.

WO 2019/236478 A1 (Guardant Health, Inc.) describes *"Methods and systems for determining the cellular origin of cell-free nucleic acids"* (title). Sukhai et al. (J Mol Diagn. 2019, 21(2):261-273) describe *"Somatic tumor variant filtration strategies to optimize tumor-only molecular profiling using targeted next-generation sequencing panels"* (title).

**SUMMARY**

The present disclosure provides methods of differentiating tumor and non-tumor origin nucleic acid variants in cell-free nucleic acid (cfNA) samples that improve the sensitivity and specificity of cancer detection assays, and guide treatment strategies, among other attributes. Additional methods as well as related systems and computer readable media are also provided. The invention is set out in the appended set of claims. References to methods for treatment of the human or animal body by surgery or therapy and diagnostic methods practised on the human or animal body are to be read as products, e.g. substances or compositions, for use in such methods.

In some aspects, the present disclosure provides a method of differentiating (e.g., distinguish between) tumor and non-tumor origin nucleic acid variants in a cell-free nucleic acid (cfNA) sample obtained from a test subject at least partially using a computer. The method includes generating or providing, by the computer, at least one tumor variant dataset comprising a population of reference tumor-related genetic variants. The tumor variant dataset comprises frequency of observance data among reference samples that comprises reference bodily fluid samples (e.g., plasma samples, serum samples, or the like) and reference non-bodily fluid samples (e.g., cell samples, tissue samples, etc.) for one or more tumor-related genetic variants in the population of reference tumor-related genetic variants. The reference samples are obtained from a single reference subject and/or from different reference subjects having an identical cancer type. The method also includes determining, by the computer, one or more ratios of the frequency of observance data between the reference samples for one or more tumor-related genetic variants in the population of reference tumor-related genetic variants to produce at least one MAF variance and/or relative prevalence dataset. In addition, the method also includes generating or providing, by the computer, at least one set of probabilities of non-tumor origin from the relative prevalence dataset, and using the set of probabilities of non-tumor origin to differentiate nucleic acid variants detected in the cfNA sample obtained from the test subject as being tumor origin nucleic acid variants or non-tumor origin nucleic acid variants. In the methods, systems, computer readable media, and other aspects of the present disclosure, one or more other features are utilized in conjunction with the ratios of the frequency of observance data. Some of these other features include uniformity of prevalence across cancer types, longitudinal mutant allele fraction (MAF) variation over time, and/or proportion in hematological cancers.

In other aspects, the present disclosure provides a method of differentiating tumor and non-tumor origin nucleic acid variants in a cell-free nucleic acid (cfNA) sample obtained from a test subject at least partially using a computer. The method includes determining, by the computer, relative prevalence of one or more tumor-related genetic variants observed in one or more reference bodily fluid samples compared to one or more reference non-bodily fluid samples to produce at least one relative prevalence dataset. In addition, the method also includes generating or providing, by the computer, at least one set of probabilities of non-tumor origin from the relative prevalence dataset, and using the set of probabilities of non-tumor origin to differentiate nucleic acid variants detected in the cfNA sample obtained from the test subject as being tumor origin nucleic acid variants or non-tumor origin nucleic acid variants.

In some aspects, the present disclosure provides a method of differentiating tumor and non-tumor origin nucleic acid variants in a cell-free nucleic acid (cfNA) sample obtained from a test subject at least partially using a computer. The method includes determining, by the computer, a variation in a mutant allele fraction (MAF) value, and/or at least one statistic related thereto (e.g., mean, standard deviation, and/or chi-square p-value of variant MAFs over time), for at least two different time points for each of one or more tumor-related and/or non-tumor-related genetic variants observed in one or more reference bodily fluid samples compared to one or more reference non-bodily fluid samples to produce at least one MAF variance and/or relative prevalence dataset. In addition, the method also includes generating or providing, by the computer, at least one set of probabilities of non-tumor origin from the MAF variance and/or relative prevalence dataset, and using the set of probabilities of non-tumor origin to differentiate nucleic acid variants detected in the cfNA sample obtained from the test subject as being tumor origin nucleic acid variants or non-tumor origin nucleic acid variants.

In some aspects, the present disclosure provides a method of differentiating tumor and non-tumor origin nucleic acid variants in a cell-free nucleic acid (cfNA) sample obtained from a test subject at least partially using a computer. The method includes classifying, by the computer, at least a first nucleic acid variant detected in the cfNA sample obtained from the test subject as being a tumor origin nucleic acid variant when a prevalence of the first nucleic acid variant detected in the cfNA sample is less than a threshold of probability from a set of probabilities of non-tumor origin and classifying, by the computer, at least a second nucleic acid variant detected in the cfNA sample obtained from the test subject as being a non-tumor origin nucleic acid variant when a prevalence of the second nucleic acid variant detected in the cfNA sample is greater than a threshold of probability from the set of probabilities of non-tumor origin, thereby differentiating the tumor and non-tumor origin nucleic acid variants in the cfNA sample obtained from the test subject. The set of probabilities of non-tumor origin is produced by: generating or providing, by the computer, at least one tumor variant dataset comprising a population of reference tumor-related genetic variants in which the tumor variant dataset comprises frequency of observance data among reference samples that comprises reference bodily fluid samples and reference non-bodily fluid samples for one or more tumor-related genetic variants in the population of reference tumor-related genetic variants, and in which the reference samples are obtained from a single reference subject and/or from different reference subjects having an identical cancer type; determining, by the computer, one or more ratios of the frequency of observance data between the reference samples for one or more tumor-related genetic variants in the population of reference tumor-related genetic variants to produce at least one relative prevalence dataset; and generating, by the computer, the set of probabilities of non-tumor origin from the relative prevalence dataset.

In other aspects, the present disclosure provides a method of producing a classifier that differentiates nucleic acid variants detected in cell-free nucleic acid (cfNA) samples as being tumor origin nucleic acid variants or non-tumor origin nucleic acid variants at least partially using a computer. The method includes generating or providing, by the computer, at least one tumor variant dataset comprising a population of reference tumor-related genetic variants, wherein the tumor variant dataset comprises frequency of observance data among reference samples that comprises reference bodily fluid samples and reference non-bodily fluid samples for one or more tumor-related genetic variants in the population of reference tumor-related genetic variants, and wherein the reference samples are obtained from a single reference subject and/or from different reference subjects having an identical cancer type. The method also includes determining, by the computer, one or more ratios of the frequency of observance data between the reference samples for one or more tumor-related genetic variants in the population of reference tumor-related genetic variants to produce at least one relative prevalence dataset. In addition, the method also includes applying, by the computer, at least one machine learning model to the relative prevalence dataset to produce at least one set of probabilities of non-tumor origin, thereby producing the classifier that differentiates the nucleic acid variants detected in the cfNA samples as being tumor origin nucleic acid variants or non-tumor origin nucleic acid variants.

In some aspects, the present disclosure provides a method of differentiating tumor and non-tumor origin nucleic acid variants in a cell-free nucleic acid (cfNA) sample obtained from a test subject having a cancer type at least partially using a computer. The method includes determining, by the computer, a prevalence of one or more genetic variants observed in the cfNA sample to produce a test subject prevalence dataset. The method also includes comparing, by the computer, the prevalence of one or more genetic variants in the test subject prevalence dataset to a prevalence of the genetic variants observed in reference cfNA samples obtained from reference subjects having the cancer type. In addition, the method includes classifying, by the computer, a given genetic variant in the test subject prevalence dataset as a non-tumor origin nucleic acid variant when the prevalence of the given genetic variant in the test subject prevalence dataset is below a predetermined threshold associated with the given genetic variant in the reference cfNA samples obtained from reference subjects having the cancer type.

In some aspects, the present disclosure provides a method of differentiating tumor and non-tumor origin nucleic acid variants in a cell-free nucleic acid (cfNA) sample obtained from a test subject at least partially using a computer. The method includes determining, by the computer, a prevalence of one or more genetic variants observed in the cfNA sample to produce a test subject prevalence dataset. The method also includes comparing, by the computer, the prevalence of one or more genetic variants in the test subject prevalence dataset to a prevalence of the genetic variants observed in reference cfNA samples obtained from reference subjects having leukemia, lymphoma, and/or hematological malignancy. In addition, the method also includes classifying, by the computer, a given genetic variant in the test subject prevalence dataset as a non-tumor origin nucleic acid variant when the prevalence of the given genetic variant in the test subject prevalence dataset is above a predetermined threshold associated with the given genetic variant in the reference cfNA samples obtained from reference subjects having the leukemia, the lymphoma, and/or the hematological malignancy.

In some embodiments, the methods disclosed herein include identifying genetic variants present in the cfNA sample from sequencing reads originating from cfNA molecules in the cfNA sample. In certain of these embodiments, the sequencing reads are obtained from targeted segments of the cfNA molecules in the cfNA sample. In some embodiments, the population of reference tumor-related genetic variants are obtained from the reference samples. In certain embodiments, the reference non-bodily fluid samples comprise reference tumor tissue samples and/or reference white blood cell samples. In some embodiments, the methods disclosed herein include obtaining the cfNA sample from the test subject. In certain embodiments, the reference samples comprise at least about 25, at least about 50, at least about 100, at least about 200, at least about 300, at least about 400, at least about 500, at least about 600, at least about 700, at least about 800, at least about 900, at least about 1,000, at least about 5,000, at least about 10,000, at least about 15,000, at least about 20,000, at least about 25,000, at least about 30,000, or more bodily fluid and/or non-bodily fluid samples. In some embodiments, the cfNA sample comprises cell-free deoxyribonucleic acid (cfDNA). In certain embodiments, the cfNA sample comprises cell-free ribonucleic acid (cfRNA). In some embodiments, the test subject is a mammalian subject. In certain embodiments, the test subject is a human subject. In some embodiments, the reference bodily fluid samples comprise plasma samples. In certain embodiments, the reference bodily fluid samples comprise serum samples. In some embodiments, the reference non-bodily fluid sample is a non-plasma sample. In some embodiments the reference non-bodily fluid (e.g., non-plasma) samples comprise cell samples. In certain embodiments, the reference non-bodily fluid (e.g., non-plasma) samples comprise tissue samples.

In some embodiments, the methods disclosed herein include selecting one or more therapies to treat a cancer type when one or more tumor origin nucleic acid variants associated with the cancer type are detected in the cfNA sample obtained from the test subject. In certain embodiments, the methods disclosed herein include administering one or more therapies to the test subject to treat a cancer type when one or more tumor origin nucleic variants associated with the cancer type are detected in the cfNA sample obtained from the test subject.

In some embodiments, the cancer type is selected from the group consisting of: bilary tract cancer, bladder cancer, transitional cell carcinoma, urothelial carcinoma, brain cancer, gliomas, astrocytomas, breast carcinoma, metaplastic carcinoma, cervical cancer, cervical squamous cell carcinoma, rectal cancer, colorectal carcinoma, colon cancer, hereditary nonpolyposis colorectal cancer, colorectal adenocarcinomas, gastrointestinal stromal tumors (GISTs), endometrial carcinoma, endometrial stromal sarcomas, esophageal cancer, esophageal squamous cell carcinoma, esophageal adenocarcinoma, ocular melanoma, uveal melanoma, gallbladder carcinomas, gallbladder adenocarcinoma, renal cell carcinoma, clear cell renal cell carcinoma, transitional cell carcinoma, urothelial carcinomas, wilms tumor, leukemia, acute lymphocytic leukemia (ALL), acute myeloid leukemia (AML), chronic lymphocytic (CLL), chronic myeloid (CML), chronic myelomonocytic (CMML), liver cancer, liver carcinoma, hepatoma, hepatocellular carcinoma, cholangiocarcinoma, hepatoblastoma, Lung cancer, non-small cell lung cancer (NSCLC), mesothelioma, B-cell lymphomas, non-Hodgkin lymphoma, diffuse large B-cell lymphoma, Mantle cell lymphoma, T cell lymphomas, non-Hodgkin lymphoma, precursor T-lymphoblastic lymphoma/leukemia, peripheral T cell lymphomas, multiple myeloma, nasopharyngeal carcinoma (NPC), neuroblastoma, oropharyngeal cancer, oral cavity squamous cell carcinomas, osteosarcoma, ovarian carcinoma, pancreatic cancer, pancreatic ductal adenocarcinoma, pseudopapillary neoplasms, acinar cell carcinomas, prostate cancer, prostate adenocarcinoma, skin cancer, melanoma, malignant melanoma, cutaneous melanoma, small intestine carcinomas, stomach cancer, gastric carcinoma, gastrointestinal stromal tumor (GIST), uterine cancer, and uterine sarcoma. In certain embodiments, the reference tumor-related genetic variants are selected from the group consisting of: single nucleotide variants (SNVs), insertions or deletions (indels), copy number variants (CNVs), fusions, transversions, translocations, frame shifts, duplications, repeat expansions, and epigenetic variants.

In some embodiments, the methods disclosed herein include randomly splitting the tumor variant dataset into a training dataset and a test dataset. In certain embodiments, the training dataset comprises about 80% of the tumor variant dataset and the test dataset comprises about 20% of the tumor variant dataset. In some embodiments, the tumor variant dataset comprises frequency of observance data among reference samples of a given cancer type for one or more tumor-related genetic variants in the population of reference tumor-related genetic variants. In some embodiments, the methods disclosed herein include training a machine learning model using at least a portion of the population of tumor-related genetic variants to produce a trained machine learning model, wherein the tumor-origin nucleic acid variants and non-tumor origin nucleic acid variants detected in the cfNA sample obtained from the test subject are differentiated from one another using the trained machine learning model. In some of these embodiments, the machine learning model is trained using one or more of: logistic regression, probit regression, decision trees, random forests, gradient boosting, support vector machines, K-nearest neighbors, and a neural network. In some embodiments, the methods disclosed herein include using a threshold of probability of at least about a 30th percentile for a given genetic variant as a cut-off for classification. In some embodiments, the methods disclosed herein include performing logistic regression on at least one of the ratios to obtain a given probability of non-tumor origin.

In some embodiments, the tumor variant dataset comprises mutant allele fraction data observed among reference samples for one or more tumor-related genetic variants in the population of reference tumor-related genetic variants. In some embodiments, the methods disclosed herein include normalizing the tumor variant dataset using one or more data normalization techniques. In certain of these embodiments, the data normalization techniques comprise min-max normalization and/or z-score normalization. In certain embodiments, a ratio of frequency of observance data of a given genetic variant in the reference bodily fluid samples relative to frequency of observance data of the given genetic variant in the reference non-bodily fluid samples that is greater than one (1.0) indicates that the given genetic variant is likely a non-tumor origin nucleic acid variant. In certain embodiments, wherein the refrence non-bodily fluid samples comprise refrence tumor tissue samples, the set of probabilities of non-tumor origin comprise at least one set of probabilities of clonal hematopoiesis origin.

In some embodiments, the tumor variant dataset comprises mutant allele fraction data observed among reference samples for one or more tumor-related genetic variants in the population of reference tumor-related genetic variants. In some embodiments, the methods disclosed herein include normalizing the tumor variant dataset using one or more data normalization techniques. In certain of these embodiments, the data normalization techniques comprise min-max normalization and/or z-score normalization. In certain embodiments, a ratio of frequency of observance data of a given genetic variant in the reference bodily fluid samples relative to frequency of observance data of the given genetic variant in the reference non-bodily fluid samples that is less than one (1.0) indicates that the given genetic variant is likely a non-tumor origin nucleic acid variant. In certain embodiments, wherein the refrence non-bodily fluid samples comprise reference white blood cell samples, the set of probabilities of non-tumor origin comprise at least one set of probabilities of clonal hematopoiesis origin.

In other aspects, the present disclosure provides a system, comprising a controller comprising, or capable of accessing, computer readable media comprising non-transitory computer-executable instructions which, when executed by at least one electronic processor perform at least: (a) generating or providing at least one tumor variant dataset comprising a population of reference tumor-related genetic variants, wherein the tumor variant dataset comprises frequency of observance data among reference samples that comprises reference bodily fluid samples and reference non-bodily fluid samples for one or more tumor-related genetic variants in the population of reference tumor-related genetic variants, and wherein the reference samples are obtained from a single reference subject and/or from different reference subjects having an identical cancer type; (b) determining one or more ratios of the frequency of observance data between the reference samples for one or more tumor-related genetic variants in the population of reference tumor-related genetic variants to produce at least one relative prevalence dataset; and (c) applying at least one machine learning model to the relative prevalence dataset to produce at least one set of probabilities of non-tumor origin to generate a classifier that differentiates the nucleic acid variants detected in cell-free nucleic acid (cfNA) samples as being tumor origin nucleic acid variants or non-tumor origin nucleic acid variants.

In other aspects, the present disclosure provides a system, comprising a controller comprising, or capable of accessing, computer readable media comprising non-transitory computer-executable instructions which, when executed by at least one electronic processor perform at least: (a) determining relative prevalence of one or more tumor-related genetic variants observed in one or more reference bodily fluid samples compared to one or more reference non-bodily fluid samples to produce at least one relative prevalence dataset; and (b) generating at least one set of probabilities of non-tumor origin from the relative prevalence dataset to generate a classifier that differentiates the nucleic acid variants detected in cell-free nucleic acid (cfNA) samples as being tumor origin nucleic acid variants or non-tumor origin nucleic acid variants.

In other aspects, the present disclosure provides a system, comprising a controller comprising, or capable of accessing, computer readable media comprising non-transitory computer-executable instructions which, when executed by at least one electronic processor perform at least: (a) determining a variation in a mutant allele fraction (MAF) value, and/or at least one statistic related thereto, for at least two different time points for each of one or more tumor-related genetic variants observed in one or more reference bodily fluid samples compared to one or more reference non-bodily fluid samples to produce at least one MAF variance and/or relative prevalence dataset; and (b) generating at least one set of probabilities of non-tumor origin from the MAF variance and/or relative prevalence dataset to generate a classifier that differentiates the nucleic acid variants detected in cell-free nucleic acid (cfNA) samples as being tumor origin nucleic acid variants or non-tumor origin nucleic acid variants.

In some embodiments, the systems disclosed herein include a nucleic acid sequencer operably connected to the controller, which nucleic acid sequencer is configured to provide sequencing reads originating from cfNA molecules in the cfNA samples. In certain of these embodiments, the nucleic acid sequencer or another system component is configured to group sequence reads generated by the nucleic acid sequencer into families of sequence reads, each family comprising sequence reads generated from a given cfNA molecule in the cfNA samples. In certain embodiments, the systems disclosed herein include a database operably connected to the controller, which database comprises one or more therapies indexed to the tumor origin nucleic acid variants. In some embodiments, the systems disclosed herein include a sample preparation component operably connected to the controller, which sample preparation component is configured to prepare the cfNA molecules in the cfNA samples to be sequenced by the nucleic acid sequencer. In certain embodiments, the systems disclosed herein include a nucleic acid amplification component operably connected to the controller, which nucleic acid amplification component is configured to amplify at least targeted segments of the cfNA molecules in the cfNA samples. In certain embodiments, the systems disclosed herein include a material transfer component operably connected to the controller, which material transfer component is configured to transfer one or more materials between at least the nucleic acid sequencer and the sample preparation component.

In some aspects, the present disclosure provides a computer readable media comprising non-transitory computer-executable instructions which, when executed by at least one electronic processor perform at least: (a) generating or providing at least one tumor variant dataset comprising a population of reference tumor-related genetic variants, wherein the tumor variant dataset comprises frequency of observance data among reference samples that comprises reference bodily fluid samples and reference non-bodily fluid samples for one or more tumor-related genetic variants in the population of reference tumor-related genetic variants, and wherein the reference samples are obtained from a single reference subject and/or from different reference subjects having an identical cancer type; (b) determining one or more ratios of the frequency of observance data between the reference samples for one or more tumor-related genetic variants in the population of reference tumor-related genetic variants to produce at least one relative prevalence dataset; and (c) applying at least one machine learning model to the relative prevalence dataset to produce at least one set of probabilities of non-tumor origin to generate a classifier that differentiates the nucleic acid variants detected in cell-free nucleic acid (cfNA) samples as being tumor origin nucleic acid variants or non-tumor origin nucleic acid variants. In the methods, systems, computer readable media, and other aspects of the present disclosure, one or more other features are utilized in conjunction with the ratios of the frequency of observance data. Some of these other features include uniformity of prevalence across cancer types, longitudinal mutant allele fraction (MAF) variation over time, and/or proportion in hematological cancers, and optionally variant gene name, position, cancer type, chromosome location, and/or the like.

In other aspects, the present disclosure provides a computer readable media comprising non-transitory computer-executable instructions which, when executed by at least one electronic processor perform at least: (a) determining relative prevalence of one or more tumor-related genetic variants observed in one or more reference bodily fluid samples compared to one or more reference non-bodily fluid samples to produce at least one relative prevalence dataset; and (b) generating at least one set of probabilities of non-tumor origin from the relative prevalence dataset to generate a classifier that differentiates the nucleic acid variants detected in cell-free nucleic acid (cfNA) samples as being tumor origin nucleic acid variants or non-tumor origin nucleic acid variants.

In other aspects, the present disclosure provides a computer readable media comprising non-transitory computer-executable instructions which, when executed by at least one electronic processor perform at least: (a) determining a variation in a mutant allele fraction (MAF) value, and/or at least one statistic related thereto, for at least two different time points for each of one or more tumor-related genetic variants observed in one or more reference bodily fluid samples compared to one or more reference non-bodily fluid samples to produce at least one MAF variance and/or relative prevalence dataset; and (b) generating at least one set of probabilities of non-tumor origin from the MAF variance and/or relative prevalence dataset to generate a classifier that differentiates the nucleic acid variants detected in cell-free nucleic acid (cfNA) samples as being tumor origin nucleic acid variants or non-tumor origin nucleic acid variants.

In some embodiments of the system or computer readable media disclosed herein, the electronic processor further performs at least: splitting (e.g., randomly or non-randomly) the tumor variant dataset into a training dataset and a test dataset. In certain embodiments of the system or computer readable media disclosed herein, the electronic processor further performs at least: training a machine learning model using at least a portion of the population of tumor-related genetic variants to produce a trained machine learning model and using the trained machine learning model differentiate the nucleic acid variants detected in the cfNA samples as being tumor origin nucleic acid variants or non-tumor origin nucleic acid variants. In some embodiments of the system or computer readable media disclosed herein, the electronic processor further performs at least: performing logistic regression on at least one of the ratios to obtain a given probability of non-tumor origin. In certain embodiments of the system or computer readable media disclosed herein, the electronic processor further performs at least: normalizing the tumor variant dataset using one or more data normalization techniques. In some embodiments of the system or computer readable media disclosed herein, the electronic processor further performs at least: comprising selecting one or more therapies to treat a cancer type when one or more tumor origin nucleic acid variants associated with the cancer type are detected in the cfNA samples.

In certain embodiments, the method, system, or computer readable media disclosed herein differentiates tumor and non-tumor origin nucleic acid variants in a cell-free nucleic acid (cfNA) sample based at least in part on: (i) the uniformity of the prevalence of the nucleic acid variant across cancer types; (ii) the variation of mutant allele fraction (MAF) of the nucleic acid variant over time; and/or (iii) the prevalence of the nucleic acid variant in hematological cancers, such as a leukemia, a lymphoma, and/or a hematological malignancy.

In some embodiments, the results of the systems and methods disclosed herein are used as an input to generate a report. The report may be in a paper or electronic format. For example, the classification that a nucleic acid variant detected in the cell-free nucleic acid sample is of a tumor or non-tumor origin, as determined by the methods and systems disclosed herein, can be displayed directly in such a report. In some embodiments, only nucleic acid variants classified as being of tumor origin are displayed in such a report.

The various steps of the methods disclosed herein, or steps carried out by the systems disclosed herein, may be carried out at the same or different times, in the same or different geographical locations, e.g. countries, and/or by the same or different people.

In other aspects, a subject may be administered a therapy based on the determination that a variant is of a tumor or non-tunor origin by the methods and systems disclosed herein. In certain embodiments, administration of a treatment to a subject may be discontinued based on the determination that a variant is of a tumor or non-tunor origin by the methods and systems disclosed herein.

## BRIEF DESCRIPTION OF THE FIGURES

FIG. 1 is a flow chart that schematically depicts exemplary method steps of differentiating tumor and non-tumor origin nucleic acid variants according to some embodiments.

FIG. 2 is a flow chart that schematically depicts exemplary method steps of differentiating tumor and non-tumor origin nucleic acid variants according to some embodiments.

FIG. 3 is a flow chart that schematically depicts exemplary method steps of differentiating tumor and non-tumor origin nucleic acid variants according to some embodiments.

FIG. 4 is an example block diagram for generating a predictive model.

FIG. 5 is a flowchart illustrating an example training method

FIG. 6 is an illustration of an exemplary process flow for using a machine learning-based classifier.

FIG. 7 is a schematic diagram of an exemplary system suitable for use with certain embodiments.

FIG. 8 panel A, are plots that show the separation in the mean standard deviation (SD) of percentages across multiple time points for non-tumor and tumor classes. FIG. 8, panel B, is a plot that shows an area under the receiver operating characteristics (ROC) curve (AUC) of a variability in longitudinal mutant allele fraction (MAF) across timepoints as a single feature in a logistic regression model. FIG. 8, panel C, is a confusion matrix table showing the accuracy of the logistic regression model.

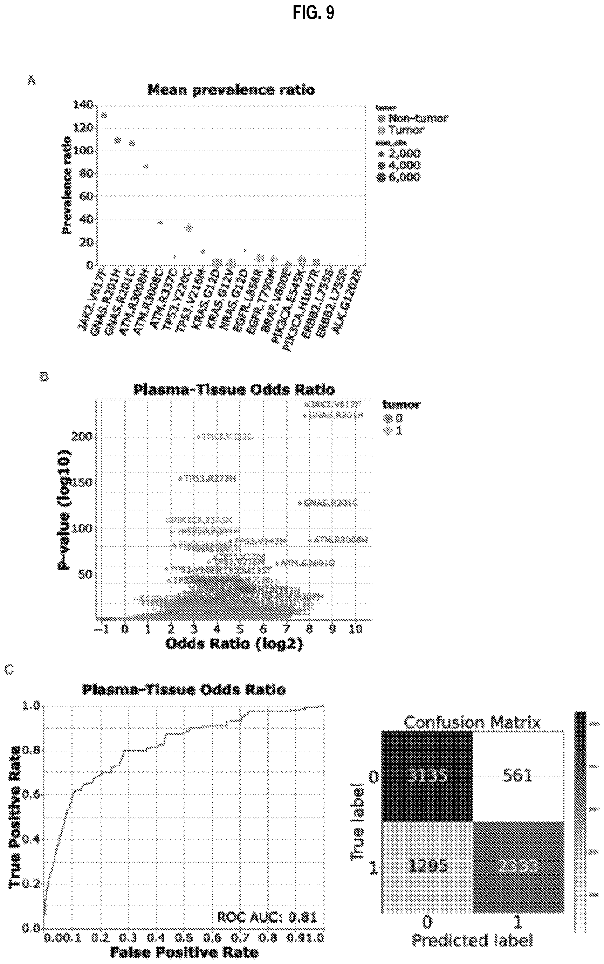

FIG. 9, panel A, is a plot of mean prevalence ratios that shows that non-tumor variants have a higher ratio of prevalence plasma compared to tumor variants, regardless of the number of clinical samples observed. Known tumor variants such as KRAS G12D and KRAS G12V have low prevalence ratio compared to JAK2 V617F, known clonal hematopoeisis variant. FIG. 9, panel B, is a volcano plot showing variants with magnitude fold-changes in plasma over tissue (x-axis) and the statistical signficiance (log10 of p-value, y-axis). Known non-tumor variants frequently observed in clonal hematopoiesis such as JAK2 V617F and GNAS R201H (blue variants, top right corner) show both large magnitude fold change as well as high statistical significance. FIG. 9, panel C, is a plot and table that table that show the performance of enrichment in plasma samples relative to tissue samples as a single feature. In particular, FIG. 9, panel C shows a ROC AUC plot and the confusion matrix table.

FIG. 10, panel A are plots that show that uniform and low prevalence is observed in non-tumor variants (top panel (panel A)) compared to known tumor variants (bottom panel (panel A)). FIG. 10, panel B shows the performance of variant prevalences across cancer types as input features into a logistic regression model. In particular, FIG. 10, panel B is a plot that shows a ROC AUC, while FIG. 10, panel C, is a confusion matrix table.

FIG. 11 (panels A and B) are a plot and table that show the performance of the proportion of samples in hematological malignancies as a single feature. In particular, FIG. 11, panel A, is a plot that shows a ROC AUC, while FIG. 11, panel B, is a confusion matrix table.

FIG. 12 schematically depicts a machine learning modeling flowchart according to some embodiments.

FIG. 13 (panels A and B) are plots and tables that show the performance of a random forest ensemble model on four input features. In particular, FIG. 13, panel A, is a ROC AUC and a confusion matrix table that show the performance of a classifier trained with a maximum (max) depth of 2 and 300 estimators. FIG. 13, panel B, is the performance of the classifier on a validation dataset with variants confirmed only in plasma (tumor) or in the white blood cell (WBC) fraction (Non-Tumor).

## DEFINITIONS

In order for the present disclosure to be more readily understood, certain terms are first defined below. Additional definitions for the following terms and other terms may be set forth throughout the specification. If a definition of a term set forth below is inconsistent with a definition in a patent application or issued patent that is incorporated by reference, the definition set forth in this application should be used to understand the meaning of the term.

As used in this specification and the appended claims, the singular forms "a," "an," and "the" include plural references unless the context clearly dictates otherwise. Thus, for example, a reference to "a method" includes one or more methods, and/or steps of the type described herein and/or which will become apparent to those persons of ordinary skill in the art upon reading this disclosure and so forth. It will also be appreciated that there is an implied "about" prior to the temperatures, concentrations, times, number of bases or base pairs, coverage, etc. discussed in the present disclosure, such that slight and insubstantial equivalents are within the scope of the present disclosure. In this application, the use of the singular includes the plural unless specifically stated otherwise. Also, the use of "comprise", "comprises", "comprising", "contain", "contains", "containing", "include", "includes", and "including" are not intended to be limiting.

It is also to be understood that the terminology used herein is for the purpose of describing particular embodiments only, and is not intended to be limiting. Further, unless defined otherwise, all technical and scientific terms used herein have the same meaning as commonly understood by one of ordinary skill in the art to which this disclosure pertains. In describing and claiming the methods, computer readable media, and systems, the following terminology, and grammatical variants thereof, will be used in accordance with the definitions set forth below.

*About:* As used herein, "about" or "approximately" as applied to one or more values or elements of interest, refers to a value or element that is similar to a stated reference value or element. In certain embodiments, the term "about" or "approximately" refers to a range of values or elements that falls within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value or element unless otherwise stated or otherwise evident from the context (except where such number would exceed 100% of a possible value or element).

*Adapter*: As used herein, "adapter" refers to short nucleic acids (e.g., less than about 500, less than about 100 or less than about 50 nucleotides in length) that are typically at least partially double-stranded and used to link to either or both ends of a given sample nucleic acid molecule. Adapters can include nucleic acid primer binding sites to permit amplification of a nucleic acid molecule flanked by adapters at both ends, and/or a sequencing primer binding site, including primer binding sites for sequencing applications, such as various next generation sequencing (NGS) applications. Adapters can also include binding sites for capture probes, such as an oligonucleotide attached to a flow cell support or the like. Adapters can also include a nucleic acid tag as described herein. Nucleic acid tags are typically positioned relative to amplification primer and sequencing primer binding sites, such that a nucleic acid tag is included in amplicons and sequencing reads of a given nucleic acid molecule. Adapters of the same or different sequence can be linked to the respective ends of a nucleic acid molecule. In certain embodiments, the same adapter is linked to the respective ends of the nucleic acid molecule except that the nucleic acid tag differs in its sequence. In some embodiments, the adapter is a Y-shaped adapter in which one end is blunt ended or tailed as described herein, for joining to a nucleic acid molecule, which is also blunt ended or tailed with one or more complementary nucleotides. In still other exemplary embodiments, an adapter is a bell-shaped adapter that includes a blunt or tailed end for joining to a nucleic acid molecule to be analyzed. Other exemplary adapters include T-tailed and C-tailed adapters.

*Administer*: As used herein, "administer" or "administering" a therapeutic agent (e.g., an immunological therapeutic agent) to a subject means to give, apply or bring the composition into contact with the subject. Administration can be accomplished by any of a number of routes, including, for example, topical, oral, subcutaneous, intramuscular, intraperitoneal, intravenous, intrathecal and intradermal.

*Allele*: As used herein, "allele" or "allelic variant" refers to a specific genetic variant at a defined genomic location or locus. An allelic variant is usually presented at a frequency of 50% (0.5) or 100%, depending on whether the allele is heterozygous or homozygous. For example, germline variants are inherited and usually have a frequency of 0.5 or 1. Somatic variants; however, are acquired variants and usually have a frequency of < 0.5. Major and minor alleles of a genetic locus refer to nucleic acids harboring the locus in which the locus is occupied by a nucleotide of a reference sequence, and a variant nucleotide different than the reference sequence respectively. Measurements at a locus can take the form of allelic fractions (AFs), which measure the frequency with which an allele is observed in a sample.

*Amplify*: As used herein, "amplify" or "amplification" in the context of nucleic acids refers to the production of multiple copies of a polynucleotide, or a portion of the polynucleotide, typically starting from a small amount of the polynucleotide (e.g., a single polynucleotide molecule), where the amplification products or amplicons are generally detectable. Amplification of polynucleotides encompasses a variety of chemical and enzymatic processes.

*Barcode*: As used herein, "barcode" in the context of nucleic acids refers to a nucleic acid molecule having a sequence that can serve as a molecular identifier. For example, individual "barcode" sequences are typically added to each DNA fragment during next-generation sequencing (NGS) library preparation so that each read can be identified and sorted before the final data analysis.

*Cancer Type*: As used herein, "cancer," "cancer type" or "tumor type" refers to a type or subtype of cancer defined, e.g., by histopathology. Cancer type can be defined by any conventional criterion, such as on the basis of occurrence in a given tissue (e.g., blood cancers, central nervous system (CNS), brain cancers, lung cancers (small cell and non-small cell), skin cancers, nose cancers, throat cancers, liver cancers, bone cancers, lymphomas, pancreatic cancers, bowel cancers, rectal cancers, thyroid cancers, bladder cancers, kidney cancers, mouth cancers, stomach cancers, breast cancers, prostate cancers, ovarian cancers, lung cancers, intestinal cancers, soft tissue cancers, neuroendocrine cancers, gastroesophageal cancers, head and neck cancers, gynecological cancers, colorectal cancers, urothelial cancers, solid state cancers, heterogeneous cancers, homogenous cancers), unknown primary origin and the like, and/or of the same cell lineage (e.g., carcinoma, sarcoma, lymphoma, cholangiocarcinoma, leukemia, mesothelioma, melanoma, or glioblastoma) and/or cancers exhibiting cancer markers, such as Her2, BRCA1, BRCA2, TP53, CA15-3, CA19-9, CA-125, CEA, AFP, PSA, HCG, KRAS, BRAF, NRAS, hormone receptor and NMP-22. Cancers can also be classified by stage (e.g., stage 1, 2, 3, or 4) and whether of primary or secondary origin.

*Cell-Free Nucleic Acid*: As used herein, "cell-free nucleic acid" or "cfNA" refers to nucleic acids not contained within or otherwise bound to a cell. Cell-free nucleic acids can include, for example, all non-encapsulated nucleic acids sourced from a bodily fluid (e.g., blood, plasma, serum, urine, cerebrospinal fluid (CSF), etc.) from a subject. Cell-free nucleic acids include DNA (cfDNA), RNA (cfRNA), and hybrids thereof, including genomic DNA, mitochondrial DNA, circulating DNA, siRNA, miRNA, circulating RNA (cRNA), tRNA, rRNA, small nucleolar RNA (snoRNA), Piwi-interacting RNA (piRNA), long non-coding RNA (long ncRNA), and/or fragments of any of these. Cell-free nucleic acids can be double-stranded, single-stranded, or a hybrid thereof. A cell-free nucleic acid can be released into bodily fluid through secretion or cell death processes, e.g., cellular necrosis, apoptosis, or the like. Some cell-free nucleic acids are released into bodily fluid from cancer cells, e.g., circulating tumor DNA (ctDNA). Others are released from healthy cells. CtDNA can be non-encapsulated tumor-derived fragmented DNA. Another example of cell-free nucleic acids is fetal DNA circulating freely in the maternal blood stream, also called cell-free fetal DNA (cffDNA). A cell-free nucleic acid can have one or more epigenetic modifications, for example, a cell-free nucleic acid can be acetylated, 5-methylated, ubiquitylated, phosphorylated, sumoylated, ribosylated, and/or citrullinated. In some embodiments, for example, the term "cell-free nucleic acid" refers to nucleic acids which are not contained within or otherwise bound to a cell at the point of isolation from a given subject.

*Cellular Origin*: As used herein, "cellular origin" or "origin" in the context of cell-free nucleic acids means the cell type from which a given cell-free nucleic acid molecule derives or otherwise originates (e.g., via a apoptotic process, a necrotic process, or the like). In certain embodiments, for example, a given cell-free nucleic acid molecule may originate from a tumor cell (e.g., a cancerous cell, etc.) or a non-tumor or normal cell (e.g., a non-cancerous cell, a hematopoietic stem cell, etc.).

*Classifier*: As used herein, "classifier" generally refers to algorithm computer code that receives, as input, test data and produces, as output, a classification of the input data as belonging to one or another class (e.g., tumor DNA or non-tumor DNA).

*Clonal Hematopoiesis-derived Mutation*: As used herein, "clonal hematopoiesis-derived mutation" or "clonal hematopoiesis origin" refers to the somatic acquisition of genomic mutations in hematopoietic stem and/or progenitor cells leading to clonal expansion.

*Clonal Hematopoiesis of Indeterminate Potential*: As used herein, "clonal hematopoiesis of indeterminate potential" or "CHIP" refers to hematopoiesis in individuals that involves the expansion of hematopoietic stem cells that comprise one or more somatic mutations (e.g., hematologic cancer-associated mutations and/or non-cancer-associated mutations), but which otherwise lack diagnostic criteria for a hematologic malignancy, such as definitive morphologic evidence of dysplasia. CHIP is a common age-related phenomenon in which hematopoietic stem cells contribute to the formation of a genetically distinct subpopulation of blood cells.

*Copy Number Variant*: As used herein, "copy number variant," "CNV," or "copy number variation" refers to a phenomenon in which sections of the genome are repeated and the number of repeats in the genome varies between individuals in the population under consideration.

*Coverage*: As used herein, "coverage" refers to the number of nucleic acid molecules that represent a particular base position.

*Deoxyribonucleic Acid or Ribonucleic Acid*: As used herein, "deoxyribonucleic acid" or "DNA" refers a natural or modified nucleotide which has a hydrogen group at the 2'-position of the sugar moiety. DNA typically includes a chain of nucleotides comprising deoxyribonucleosides that each comprise one of four types of nucleobases, namely, adenine (A), thymine (T), cytosine (C), and guanine (G). As used herein, "ribonucleic acid" or "RNA" refers to a natural or modified nucleotide which has a hydroxyl group at the 2'-position of the sugar moiety. RNA typically includes a chain of nucleotides comprising ribonucleosides that each comprise one of four types of nucleobases, namely, A, uracil (U), G, and C. As used herein, the term "nucleotide" refers to a natural nucleotide or a modified nucleotide. Certain pairs of nucleotides specifically bind to one another in a complementary fashion (called complementary base pairing). In DNA, adenine (A) pairs with thymine (T) and cytosine (C) pairs with guanine (G). In RNA, adenine (A) pairs with uracil (U) and cytosine (C) pairs with guanine (G). When a first nucleic acid strand binds to a second nucleic acid strand made up of nucleotides that are complementary to those in the first strand, the two strands bind to form a double strand. As used herein, "nucleic acid sequencing data," "nucleic acid sequencing information," "sequence information," "nucleic acid sequence," "nucleotide sequence", "genomic sequence," "genetic sequence," or "fragment sequence," or "nucleic acid sequencing read" denotes any information or data that is indicative of the order and identity of the nucleotide bases (e.g., adenine, guanine, cytosine, and thymine or uracil) in a molecule (e.g., a whole genome, whole transcriptome, exome, oligonucleotide, polynucleotide, or fragment) of a nucleic acid such as DNA or RNA. It should be understood that the present teachings contemplate sequence information obtained using all available varieties of techniques, platforms or technologies, including, but not limited to: capillary electrophoresis, microarrays, ligation-based systems, polymerase-based systems, hybridization-based systems, direct or indirect nucleotide identification systems, pyrosequencing, ion- or pH-based detection systems, and electronic signature-based systems.

*Detect*: As used herein, "detect," "detecting," or "detection" refers to an act of determining the existence or presence of one or more target nucleic acids (e.g., nucleic acids having targeted mutations or other markers) in a sample.

*Hematopoietic Stem Cell*: As used herein, "hematopoietic stem cell" or "HSC" is a stem cell that gives rise to other blood cells through the process of hematopoiesis.

*Indel*: As used herein, "indel" refers to mutation that involves the insertion or deletion of nucleotide positions in the genome of a subject.

*Indexed*: As used herein, "indexed" refers to a first element (e.g., clinical information) linked to a second element (e.g., a given sample, a recommended therapy, etc.).

*Machine Learning Algorithm*: As used herein, "machine learning algorithm" generally refers to an algorithm, executed by computer, that automates analytical model building, e.g., for clustering, classification or pattern recognition. Machine learning algorithms may be supervised or unsupervised. Learning algorithms include, for example, artificial neural networks (e.g., back propagation networks), discriminant analyses (e.g., Bayesian classifier or Fisher's analysis), support vector machines, decision trees (e.g., recursive partitioning processes such as CART - classification and regression trees, or random forests), linear classifiers (e.g., multiple linear regression (MI,R), partial least squares (PLS) regression, and principal components regression), hierarchical clustering, and cluster analysis. A dataset on which a machine learning algorithm learns can be referred to as "training data." A model produced using a machine learning algorithm is generally referred to herein as a "machine learning model."

*Minor Allele Frequency*: As used herein, "minor allele frequency" refers to the frequency at which minor alleles (e.g., not the most common allele) occurs in a given population of nucleic acids, such as a sample obtained from a subject. Genetic variants at a low minor allele frequency typically have a relatively low frequency of presence in a sample.

*Mutant Allele Fraction*: As used herein, "mutant allele fraction," or "MAF" refers to the fraction of nucleic acid molecules harboring an allelic alteration or mutation with respect to a reference at a given genomic position in a given sample. MAF is generally expressed as a fraction or percentage. For example, MAF is typically less than about 0.5, 0.1, 0.05, or 0.01 (i.e., less than about 50%, 10%, 5%, or 1%) of all somatic variants or alleles present at a given locus.

*Mutation*: As used herein, "mutation," "nucleic acid variant," "variant," or "genetic aberration" refers to a variation from a known reference sequence and includes mutations such as, for example, single nucleotide variants (SNVs), copy number variants or variations (CNVs)/aberrations, insertions or deletions (indels), truncation, gene fusions, transversions, translocations, frame shifts, duplications, repeat expansions, and epigenetic variants. A mutation can be a germline or somatic mutation. In some embodiments, a reference sequence for purposes of comparison is a wildtype genomic sequence of the species of the subject providing a test sample, typically the human genome. In certain cases, a mutation or variant is a "tumor-related genetic variant" that causes or at least contributes to oncogenesis.

*Next Generation Sequencing*: As used herein, "next generation sequencing" or "NGS" refers to sequencing technologies having increased throughput as compared to traditional Sanger- and capillary electrophoresis-based approaches, for example, with the ability to generate hundreds of thousands of relatively small sequence reads at a time. Some examples of next generation sequencing techniques include, but are not limited to, sequencing by synthesis, sequencing by ligation, and sequencing by hybridization.

*Nucleic Acid Tag*: As used herein, "nucleic acid tag" refers to a short nucleic acid (e.g., less than about 500, about 100, about 50 or about 10 nucleotides in length), used to label nucleic acid molecules to distinguish nucleic acids from different samples (e.g., representing a sample index), or different nucleic acid molecules in the same sample (e.g., representing a molecular tag), of different types, or which have undergone different processing. Nucleic acid tags can be single stranded, double stranded or at least partially double stranded. Nucleic acid tags optionally have the same length or varied lengths. Nucleic acid tags can also include double-stranded molecules having one or more blunt-ends, include 5' or 3' single-stranded regions (e.g., an overhang), and/or include one or more other single-stranded regions at other locations within a given molecule. Nucleic acid tags can be attached to one end or both ends of the other nucleic acids (e.g., sample nucleic acids to be amplified and/or sequenced). Nucleic acid tags can be decoded to reveal information such as the sample of origin, form or processing of a given nucleic acid. Nucleic acid tags can also be used to enable pooling and/or parallel processing of multiple samples comprising nucleic acids bearing different nucleic acid tags and/or sample indexes in which the nucleic acids are subsequently being deconvoluted by reading the nucleic acid tags. Nucleic acid tags can also be referred to as molecular identifiers or tags, sample identifiers, index tags, and/or barcodes. Additionally or alternatively, nucleic acid tags can be used to distinguish different molecules in the same sample. This includes, for example, uniquely tagging each different nucleic acid molecule in a given sample, or non-uniquely tagging such molecules. In the case of non-unique tagging applications, tags with a limited number of different sequences may be used to tag each nucleic acid molecule such that different molecules can be distinguished based on, for example, start and/or stop positions where they map to a selected reference genome in combination with at least one nucleic acid tag. Typically, a sufficient number of different nucleic acid tags are used such that there is a low probability (e.g., less than about a 10%, less than about a 5%, less than about a 1%, or less than about a 0.1% chance) that any two molecules will have the same start/stop positions and also have the same nucleic acid tag. Some nucleic acid tags include multiple molecular identifiers to label samples, forms of nucleic acid molecules within a sample, and nucleic acid molecules within a form having the same start and stop positions. Such nucleic acid tags can be referenced using the exemplary form "A1i" in which the uppercase letter indicates a sample type, the Arabic numeral indicates a form of molecule within a sample, and the lowercase Roman numeral indicates a molecule within a form.

*Polynucleotide*: As used herein, "polynucleotide", "nucleic acid", "nucleic acid molecule", or "oligonucleotide" refers to a linear polymer of nucleosides (including deoxyribonucleosides, ribonucleosides, or analogs thereof) joined by internucleosidic linkages. Typically, a polynucleotide comprises at least three nucleosides. Oligonucleotides often range in size from a few monomeric units, e.g. 3-4, to hundreds of monomeric units. Whenever a polynucleotide is represented by a sequence of letters, such as "ATGCCTG," it will be understood that the nucleotides are in 5' → 3' order from left to right and that in the case of DNA, "A" denotes deoxyadenosine, "C" denotes deoxycytidine, "G" denotes deoxyguanosine, and "T" denotes deoxythymidine, unless otherwise noted. The letters A, C, G, and T may be used to refer to the bases themselves, to nucleosides, or to nucleotides comprising the bases, as is standard in the art.

*Prevalence*: As used herein, "prevalence" or "frequency of observance" in the context of nucleic acid variants refers to the degree, pervasiveness, or frequency with which a given nucleic acid variant is or was observed in a given sample (e.g., a given bodily fluid sample, a given non-bodily fluid sample, etc.) or other population (e.g., a given population of bodily fluid samples, a given population of non-bodily fluid samples, etc.).

*Reference Sample*: As used herein, "reference sample" or "reference cfNA sample" refers a sample of known composition and/or having or known to have or lack specific properties (e.g., known nucleic acid variant(s), known cellular origin, known tumor fraction, known coverage, and/or the like) that is analyzed along with or compared to test samples in order to evaluate the accuracy of an analytical procedure, classify the test samples, and/or the like. A reference sample dataset typically includes from at least about 25 to at least about 30,000 or more reference samples. In some embodiments, the reference sample dataset includes about 50, 75, 100, 150, 200, 300, 400, 500, 600, 700, 800, 900, 1,000, 2,500, 5,000, 7,500, 10,000, 15,000, 20,000, 25,000, 50,000, 100,000, 1,000,000, or more reference samples.

*Reference Sequence*: As used herein, "reference sequence" or "reference genome" refers to a known sequence used for purposes of comparison with experimentally determined sequences. For example, a known sequence can be an entire genome, a chromosome, or any segment thereof. A reference sequence typically includes at least about 20, at least about 50, at least about 100, at least about 200, at least about 250, at least about 300, at least about 350, at least about 400, at least about 450, at least about 500, at least about 1000, at least about 100000, at least about 1000000, at least about 1000000000, or more nucleotides. A reference sequence can align with a single contiguous sequence of a genome or chromosome or can include non-contiguous segments that align with different regions of a genome or chromosome. Exemplary reference sequences, include, for example, human genomes, such as, hG19 and hG38.

*Sample*: As used herein, "sample" means any biological sample capable of being analyzed by the methods and/or systems disclosed herein. In certain aspects of the present disclosure, samples are bodily fluid samples, for example, whole blood or fractions thereof, lymphatic fluid, urine, and/or cerebrospinal fluid, among other bodily fluid types from which cell-free (circulating, not contained within or otherwise bound to a cell) nucleic acids are sourced. In certain implementations, bodily fluid samples are plasma samples, which are the fluid portions of whole blood exclusive of cells, such as red and white blood cells. In some implementations, bodily fluid samples are serum samples, that is, plasma lacking fibrinogen. In some aspects of the present disclosure, samples are "non-bodily fluid samples" or "non-plasma samples," that is, biological samples other than "bodily fluid samples" such as, as cellular and/or tissue samples, from which nucleic acids other than cell-free nucleic acids are sourced.

*Sensitivity*: As used herein, "sensitivity" in the context of a given assay or method refers to the ability of the assay or method to detect and distinguish between targeted (e.g., nucleic acid variants) and non-targeted analytes.

*Sequencing*: As used herein, "sequencing" refers to any of a number of technologies used to determine the sequence (e.g., the identity and order of monomer units) of a biomolecule, e.g., a nucleic acid such as DNA or RNA. Exemplary sequencing methods include, but are not limited to, targeted sequencing, single molecule real-time sequencing, exon or exome sequencing, intron sequencing, electron microscopy-based sequencing, panel sequencing, transistor-mediated sequencing, direct sequencing, random shotgun sequencing, Sanger dideoxy termination sequencing, whole-genome sequencing, sequencing by hybridization, pyrosequencing, capillary electrophoresis, duplex sequencing, cycle sequencing, single-base extension sequencing, solid-phase sequencing, high-throughput sequencing, massively parallel signature sequencing, emulsion PCR, co-amplification at lower denaturation temperature-PCR (COLD-PCR), multiplex PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD™ sequencing, MS-PET sequencing, and a combination thereof. In some embodiments, sequencing can be performer by a gene analyzer such as, for example, gene analyzers commercially available from Illumina, Inc., Pacific Biosciences, Inc., or Applied Biosystems/Thermo Fisher Scientific, among many others.

*Sequence Information*: As used herein, "sequence information" in the context of a nucleic acid polymer means the order and identity of monomer units (e.g., nucleotides, etc.) in that polymer.

*Single Nucleotide Variant*: As used herein, "single nucleotide variant" or "SNV" means a mutation or variation in a single nucleotide that occurs at a specific position in the genome.

*Somatic Mutation*: As used herein, "somatic mutation" means a mutation in the genome that occurs after conception. Somatic mutations can occur in any cell of the body except germ cells and accordingly, are not passed on to progeny.

*Specificity*: As used herein, "specificity" in the context of a diagnostic analysis or assay refers to the extent to which the analysis or assay detects an intended target analyte to the exclusion of other components of a given sample.

*Subject*: As used herein, "subject" or "test subject" refers to an animal, such as a mammalian species (e.g., human) or avian (e.g., bird) species, or other organism, such as a plant. More specifically, a subject can be a vertebrate, e.g., a mammal such as a mouse, a primate, a simian or a human. Animals include farm animals (e.g., production cattle, dairy cattle, poultry, horses, pigs, and the like), sport animals, and companion animals (e.g., pets or support animals). A subject can be a healthy individual, an individual that has or is suspected of having a disease or a predisposition to the disease, or an individual that is in need of therapy or suspected of needing therapy. The terms "individual" or "patient" are intended to be interchangeable with "subject." In some embodiments, the subject is a human who has, or is suspected of having cancer. For example, a subject can be an individual who has been diagnosed with having a cancer, is going to receive a cancer therapy, and/or has received at least one cancer therapy. The subject can be in remission of a cancer. As another example, the subject can be an individual who is diagnosed of having an autoimmune disease. As another example, the subject can be a female individual who is pregnant or who is planning on getting pregnant, who may have been diagnosed with or suspected of having a disease, e.g., a cancer, an auto-immune disease.

*Threshold*: As used herein, "threshold" refers to a separately determined value used to characterize or classify experimentally determined values. In certain embodiments, for example, "threshold" refers to a selected value to which a quantitative value is compared in order to determine that a given nucleic acid variant is a tumor origin nucleic acid variant or a non-tumor origin nucleic acid variant. In some of these embodiments, the selected value is a "threshold of probability"

*Tumor Fraction*: As used herein, "tumor fraction" refers to the estimate of the fraction of nucleic acid molecules derived from tumor in a given sample. For example, the tumor fraction of a sample can be a measure derived from the maximum mutant allele fraction (MAX MAF) of the sample or coverage of the sample, or length, epigenetic state, or other properties of the cfNA fragments in the sample or any other selected feature of the sample. The term "MAX MAF" refers to the maximum or largest MAF of all somatic variants present in a given sample. In some embodiments, the tumor fraction of a sample is equal to the MAX MAF of the sample.

*Value*: As used herein, "value" generally refers to an entry in a dataset that can be anything that characterizes the feature to which the value refers. This includes, without limitation, numbers, words or phrases, symbols (e.g., + or -) or degrees.

## DETAILED DESCRIPTION

Tumor-derived somatic variants in circulating nucleic acids, such as cell-free DNA (cfDNA), can be used for targeted therapy selection, longitudinal monitoring, and early detection of cancer. Cell-free tumor DNA (ctDNA) are small DNA fragments released from necrotic/apoptotic tumor cells or circulating tumor cells (CTCs) into the bloodstream. The vast majority of cfDNA is derived from normal cells, including normal leukocytes that undergo apoptosis or necrosis. Recent studies demonstrate that a significant proportion of mutations detected in the cfDNA can originate from non-tumor sources, particularly from clonal hematopoiesis, which results in the accumulation of somatic mutations in hematopoietic stem cells, contributing to the cfDNA 'noise' (Razavi et al., "High-intensity sequencing reveals the sources of plasma circulating cell-free DNA variants," Nature Medicine, 25:1928-1937 (2019)). The presence of non-tumor variants in the plasma/cfDNA can confound ctDNA interpretation; therefore, methods and related aspects of differentiating these is highly sought.

Current approaches to identifying nucleic acid variants that derive or otherwise originate from clonal hematopoiesis from cancer tumor nucleic acid variants, include sequencing white blood cells (WBC) or peripheral blood mononuclear cells and removing these sequences from the nucleic acid variants in the plasma portion of a given blood sample, sequencing tissue and removing all nucleic acid variants exclusive of tissue in plasma fractions, or a combination of both techniques (Id.). Bioinformatic approaches that have been attempted include removing nucleic acid variants occurring in genes frequently mutated in hematological malignancies (Coombs et al., "Therapy-related clonal hematopoiesis in patients with non-hematologic cancers is common and impacts clinical outcome," Cell Stem Cell, 21(3):374-382 (2017)), as they are likely to originate from the hematological fraction, comparing nucleic acid fragment sizes for a single locus in the cfDNA of wild-type and WBC (Hubbell et al, "Cell-free DNA (cfDNA) fragment length patterns of tumor- and blood-derived variants in participants with and without cancer," 2019 AACR Meeting, Abstract 3372, March 29-April 3, 2019), and using absolute or relative variant minor allele frequency cut-offs with respect to the tumor (Li et al., "Ultra-deep next generation sequencing of plasma cell-free DNA in patients with advanced lung cancers: results from the Actionable Genome Consortium," Ann. Oncol., 30(4):597-603 (2019)). The challenges of these approaches lie in the requirement of matched WBC and tissue, which is not always available and complicates sample processing. The present disclosure presents novel bioinformatics methods and related aspects to classify nucleic acid variants or mutations detected in plasma or other bodily fluids as being from tumor or non-tumor, independent of the availability of matched WBC or tumor tissue.

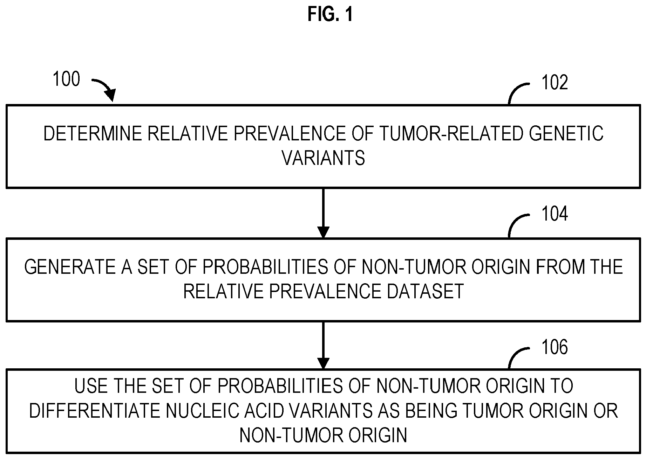

FIG. 1 is a flow chart that schematically depicts exemplary method steps of differentiating tumor and non-tumor origin nucleic acid variants in a cell-free nucleic acid (cfNA) sample obtained from a test subject according to some embodiments. For example, the methods disclosed herein can be used to facilitate the removal or reduction of background noise created by non-tumor origin nucleic acid variants (e.g., cfDNA fragments originating from non-cancerous or normal cells) detected in a given sample from a test subject to thereby improve assay sensitivity. As shown, method 100 includes determining (e.g., by a computer) relative prevalence of tumor-related genetic variants observed in reference bodily fluid samples (e.g., plasma samples, serum samples, or the like) compared to reference non-bodily fluid samples (e.g., cell samples, tissue samples, or the like) to produce a relative prevalence dataset (step 102). Method 100 also includes generating (e.g., by a computer) a set of probabilities of non-tumor origin from the relative prevalence dataset (step 104). In addition, method 100 further includes using the set of probabilities of non-tumor origin to differentiate nucleic acid variants detected in the cfNA sample obtained from the test subject as being tumor origin nucleic acid variants or non-tumor origin nucleic acid variants (step 106). Related systems and computer readable media for implementing the methods disclosed herein are further described below.

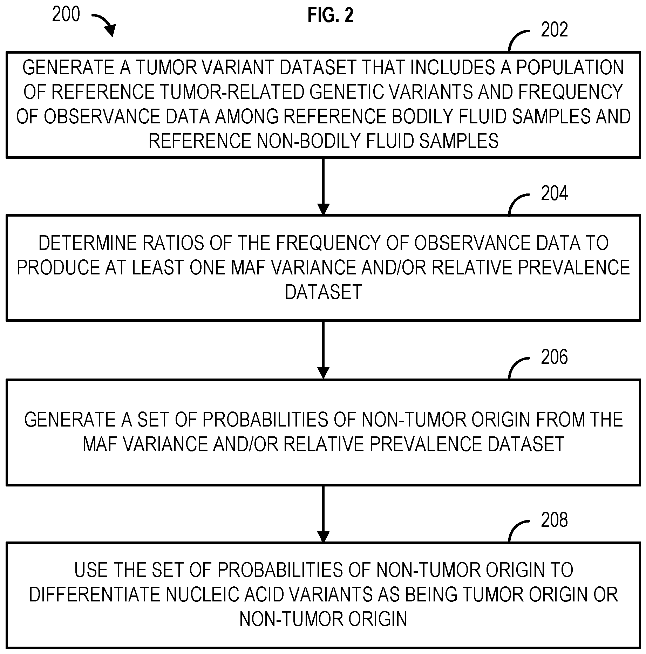

To further illustrate, FIG. 2 is a flow chart that schematically depicts exemplary method steps of differentiating tumor and non-tumor origin nucleic acid variants in a cell-free nucleic acid (cfNA) sample obtained from a test subject according to some embodiments. As shown, method 200 includes generating (e.g., by a computer) a tumor variant dataset that includes a population of reference tumor-related genetic variants in which the tumor variant dataset includes frequency of observance (prevalence) data among reference samples that include reference bodily fluid samples (e.g., plasma samples, serum samples, or the like) and/or reference non-bodily fluid samples (e.g., cell samples, tissue samples, or the like) for tumor-related genetic variants in the population of reference tumor-related genetic variants (step 202). The reference samples are typically obtained from a single reference subject and/or from different reference subjects having an identical cancer type. Method 200 also includes determining (e.g., by a computer) ratios of the frequency of observance data between the reference samples for tumor-related genetic variants in the population of reference tumor-related genetic variants to produce at least one MAF variance and/or relative prevalence dataset (step 204). Method 200 further includes generating (e.g., by a computer) a set of probabilities of non-tumor origin from the MAF variance and/or relative prevalence dataset (step 206). In addition, method 200 also includes using the set of probabilities of non-tumor origin to differentiate nucleic acid variants detected in the cfNA sample obtained from the test subject as being tumor origin nucleic acid variants or non-tumor origin nucleic acid variants (step 208).

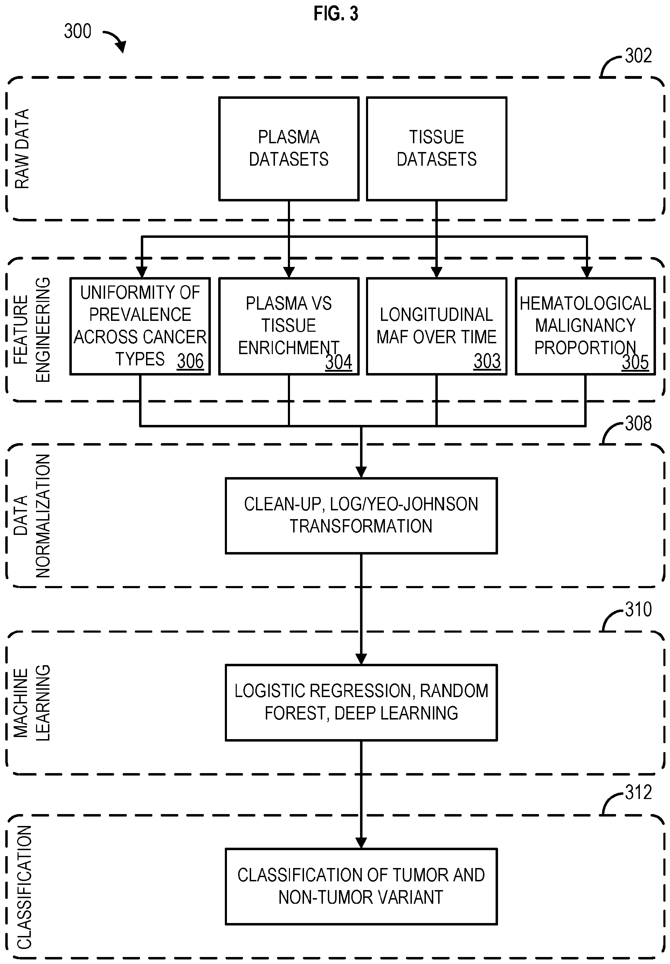

As an additional illustration, FIG. 3 is a flow chart that schematically depicts exemplary method steps of differentiating or classifying tumor and non-tumor origin nucleic acid variants in a cell-free nucleic acid (cfNA) sample obtained from a test subject according to some embodiments. As shown, method 300 includes obtaining raw data, for example, in the form of cancer and non-cancer (i.e., normal or healthy) sample data and tissue sample data (e.g., from the COSMIC Cancer Database, The Cancer Genome Atlas (TCGA) data, Memorial Sloan Kettering Cancer Center (MSKCC) data, and/or another data source) (step 302). In a feature engineering step, input features are created by, for example, calculating mutant allele fraction (MAF) variations over time (step 303), calculating raw numbers and prevalences nucleic acid variants for all cancer types and calculating ratios between prevalences of nucleic acid variants observed in plasma and/or other bodily fluids and tissue datasets for all cancer types (step 304), calculating the proportion of nucleic acid variants in hematological malignancies or other cancer types (step 305), and testing for uniformity (e.g., developing uniformity scores) across cancer types for plasma and/or other bodily fluids sample prevalences (step 306). The bioinformatic data may include frequency of observance of a genetic variant among samples of particular cancer type, including hematological malignancies; prevalence of variants in plasma and/or other bodily fluids, tumor tissue, white blood cells, mutant allele fraction of a variant, and others. Additional or other data types are optionally used for these feature engineering steps. Method 300 also includes transformation and clean-up processes, such as, clean-up for sample prevalences (e.g., adjust for samples with a low number of a given nucleic acid variant, low number of samples, etc.), perform log transformations (e.g., Log (x + 1) or Np.log1p), and perform normalization (e.g., Yeo-Johnson normalization, min-max normalization, z-score normalization, and/or the like) (step 308). Method 300 also includes a machine learning step that generates a machine learning model to provide probabilities of non-tumor nucleic acid variants being present in a given sample using, for example, logistic regression or a deep learning technique (step 310). Exemplary models that can be used for training and further classification, without limitations, include logistic regression, probit regression, decision trees, random forests, gradient boosting, support vector machines, k-nearest neighbors, neural networks, or an ensemble of more than one of these methods. Ensemble methods are meta-algorithms that combine several machine learning techniques into one predictive model in order to decrease variance (bagging), bias (boosting), or improve predictions (stacking). Most ensemble methods use a single base learning algorithm to produce homogeneous base learners, that is, learners of the same type, leading to homogeneous ensembles. There are also some methods that use heterogeneous learners, that is, learners of different types, leading to heterogeneous ensembles. In order for ensemble methods to be more accurate than any of its individual members, the base learners have to be as accurate as possible and as diverse as possible.

Datasets are optionally split into training and test sets using various approaches. In some embodiments, for example, datasets are randomly split into training and test datasets with an 80/20 proportion. In addition method 300 also includes selecting a cut-off value for determining a threshold for classifying nucleic acid variants as being tumor or non-tumor cell origin (step 312).

Bodily Fluid:Tissue Ratio - Binary Classification

Some embodiments include comparing prevalences of variants observed in bodily fluid sample (e.g., plasma sample) datasets relative to their occurrence in tissue datasets of the same cancer origin. In certain of these embodiments, logistic regression is performed on these ratios to obtain probabilities of clonal hematopoiesis origin.

In some embodiments, values of the performance metrics may include, for example, accuracy (i.e., fraction of correct predictions), balanced_accuracy (defined as the average of recall obtained on each class), precision_macro (involves calculating metrics for each label, and then finding their unweighted mean; but, this approach does not take label imbalance into account), precision_micro (involves calculating metrics globally by counting the total true positives, false negatives and false positives), precision_weighted (involves calculating metrics for each label and finding their average weighted by support (e.g., to determine the number of true instances for each label)), and the like. In certain embodiments, performance metrics are estimated by stratified 5-fold cross-validation on the training set (e.g., in which the folds are made by preserving the percentage of samples for each class).

Box-Cox transformation is optionally used to transform non-normal distributions to normal distributions, but this approach does not work with negative numbers. In contrast, Yeo-Johnson transformation allows one to work with negative numbers. For example, for both logistic regression and support vector machine (SVM) models, all features are optionally first transformed with Yeo-Johnson transform (parametric, monotonic transformation that is applied to make data more Gaussian-like in order to stabilize variance and minimize skewness). The Yeo-Johnson transform is given by Equation 1:

$$x_i^{(\lambda)} = \begin{cases} [(x_i + 1)^\lambda - 1]/\lambda & \text{if } \lambda \neq 0, x_i \geq 0, \\ \ln(x_i) + 1 & \text{if } \lambda = 0, x_i \geq 0 \\ -[(-x_i + 1)^{2-\lambda} - 1]/(2 - \lambda) & \text{if } \lambda \neq 2, x_i < 0, \\ -\ln(-x_i + 1) & \text{if } \lambda = 2, x_i < 0 \end{cases}$$

(Equation 1)

where $x_i$ is i-th data point in a dataset and $\lambda$ is the power parameter. $\lambda$ can be any real number, where $\lambda=1$ produces the identity transformation. In some embodiments, zero-mean, unit-variance normalization is further applied to the transformed data.

In some embodiments, the basic inputs used to define the set of parameters are: (1) model type, and (2) set of hyperparameters. In certain embodiments, the resulting parameters are used for all future classification. In some embodiments, the training set is used to run grid search with 5-fold stratified cross-validation over the following sets of hyperparameters (e.g. to define the cost of misclassification): kernel: linear, C: [0.001, 0.01, 0.1, 1, 10, 100, 1000], and kernel: radial basis function (rbf), C: [0.001, 0.01, 0.1, 1, 10, 100, 1000], gamma: [0.0001, 0.001, 0.01, 0.1, 0.2, 0.3, 0.4, 0.5, 1].

Direct training on dataset

Some embodiments use machine learning and features about a variant gene name, position, cancer type, chromosome location and other features based on known datasets to predict tumor/non-tumor origin. In these embodiments, the method typically includes training a machine learning model on clonal hematopoiesis (CH) and tissue specific training datasets to identify features specific to either origin, and applying the model to historical variants observed in a previous dataset to determine the probability that a given variant can be attributed to CH. In these embodiments, the method also typically includes determining which threshold of probability is optimal for accurate classification of CH, and applying this list of probabilities to a new dataset in order to classify its origin as tumor or clonal hematopoiesis. In certain of these embodiments, the top 10th percentile of variants will be a small number of variants, but will have high predictive value of being CH in origin.

Prevalence in bodily fluid relative to tumor tissue

Certain embodiments use higher prevalence of a given variant in bodily fluid (e.g., plasma or serum) databases relative to their occurrence in tumor tissue, where clonal hematopoiesis (CH) may be less confounding, and thus, may inform on variants that are likely to come from CH. In some of these embodiments, the method includes determining prevalences of specific variants occurring in a bodily fluid database and comparing those to prevalences observed in primary tissue databases, such as the COSMIC database or the like. Some of these embodiments include determining ratios of prevalence of variants observed in bodily fluid samples to prevalence of those variants observed in tissue samples. Some of these embodiments include calculating the odds ratio for the prevalence of variants and the probability that the value of the odds ratio is equal, greater or less than 1. In these embodiments, the method also generally includes applying a machine learning model to these relative prevalence values of bodily fluid versus tissue prevalences to determine the probability that the variant is likely to originate from CH or non-tumor status. A threshold of probability (e.g. about 10th, 15th, 20th, 25th, 30th, 35th, 40th, or another percentile) is typically used as a cut-off for classification. Generally, a small number of variants will have high predictive value of being non-tumor or CH.

Multi-class model using distribution across tumor types or uniformity test

In some embodiments, tumor specific variants will have a particular distribution or selection depending on the biology of each cancer type. If the variant is not specific to a cancer type, it will typically have a uniform distribution, which may be indicative of a passenger mutation or non-tumor status. Accordingly, certain methods determine prevalences of variants across tumor types, or their relative proportions and representations, which a machine learning model could be trained to separate into distinct tumor and non-tumor classes. Some of these methods include using Coefficients of Variation to determine distributions and any significant enrichment in specific tumor types. In certain of these embodiments, a very small number of variants will be predictive and specific to a tumor type and unlikely to be CH. Some variants will have no demonstrative selectivity to a particular tumor type and low prevalence across all tumor types, indicating likely CH. Generally, if a variant is substantially uniform across tumor types, then it is likely non-tumor/CH in origin, whereas if a variant is highly prevalent in certain tumors, then it is more likely there is a biological selection for that variant in the tumor. Current methods that rely strictly on patient age or absolute VAF, and methods that ignore the expected relative prevalence in different tumor settings will fail to consider these underlying disease-specific mechanisms (or absence there-of) driving the observed VAF and key biological features indicating the variant origin.
Other input features to the machine learning model in these embodiments, include, for example, tumor classification based on variants (e.g. tumor type, or expected tumor type), methylation presence or signatures, other variants within a given sample (e.g., known CH variants present in the sample, increasing the probability of other variants in the sample are also non-tumor in origin), difference in family size for a given variant versus a reference allele, the nature of the observed nucleotide or other change in the variant, the absolute value of MAF within the sample, the relative value of MAF within the sample, how the value of the MAF within the sample changes over time relative to other variants, and/or the like.

Monitor value of MAF changes over time

In certain cases, clones of variants of non-tumor variants will likely remain more stable in a subject over time compared to variants originating from the tumor. Accordingly, in some embodiments, the method includes for each patient with multiple time points (e.g. >3), calculating the Coefficient of Variation (CV, dispersion relative to the mean) of the variant percentage over time, and computing the statistics and distributions of CV across all variants and patients. In these embodiments, known driver or tumor variants will generally have dynamic percentages over time (due to tumor growth and shrinkage), with large CV across time points compared to non-tumor variants. This can also be used as an input feature for the classifier. In contrast, the non-tumor variant MAF is typically less dynamic and more stable over time compared to true tumor variants, and would have a lower CV over time. The distribution of these CVs can be separated in a machine learning model and provide a robust classification of tumor or non-tumor status. Other input features to the machine learning model in these embodiments, include, for example, variant clonality (relative VAF to the tumor fraction) over time or across patients, fragmentomics data points, fragment size, position, age of the patient (older patients have higher probability of CHIP), and/or the like. Current methods that may track VAF or VAF dispersion across timepoints in a single patient will less accurate than an approach that aggregates the VAFs across patients in mass, particularly if these patients were all serially tested on the same platform and bioinformatics pipelines leading to consistent VAFs and more robust measurements of variation. Further, this method of classification may use static thresholds that do not adjust for the value of dispersion relative to the absolute VAFs, where non-tumor variants of higher VAFs may be confounded with lower VAF variants that have similar measurements of dispersion over time. A machine learning model that takes into account both the absolute VAFs as well as the VAF dispersion across timepoints in a sufficiently large cohort of patients measured on the same platform will have higher resolution for classification and be less likely to result in false positive or negative labeling of tumor/non-tumor status.

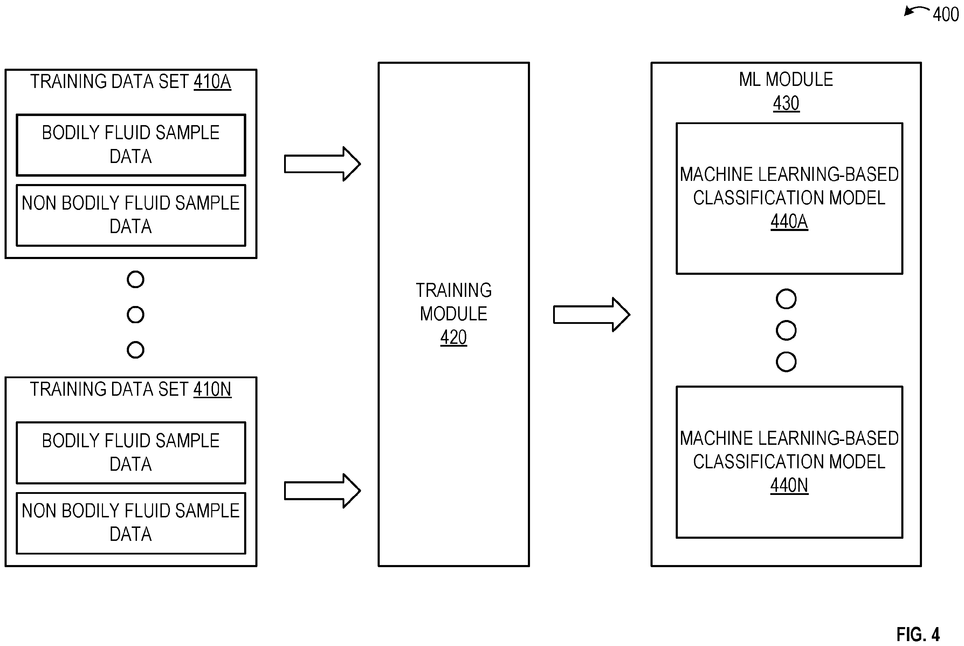

Turning now to FIG. 4, additional methods are described for generating a predictive model (e.g,. classification model). The methods described may use machine learning ("ML") techniques to train, based on an analysis of one or more training data sets 410A-410N by a training module 420, at least one ML module 430 that is configured to classify mutations detected in plasma as tumor origin or non-tumor origin, which can be from clonal hematopoiesis or biological noise.

The one or more training data sets 410A-410N may comprise cancer/non-cancer (e.g., tumor/non-tumor) bodily fluid (e.g., blood, plasma, serum, cerebrospinal fluid, urine) sample data and cancer/non-cancer (e.g., tumor/non-tumor) non-bodily fluid (e.g., tissue) sample data (e.g., from the COSMIC Cancer Database, The Cancer Genome Atlas (TCGA) data, and/or another data source). A subset of the cancer/non-cancer bodily fluid sample data and/or the cancer/non-cancer non-bodily fluid sample data may be randomly assigned to the training data set 410 or to a testing data set. In some implementations, the assignment of data to a training data set or a testing data set may not be completely random. In this case, one or more criteria may be used during the assignment. In general, any suitable method may be used to assign the data to the training or testing data sets, while ensuring that the data distributions are somewhat similar in the training data set and the testing data set.

The training module 420 may train the ML module 430 by extracting a feature set from the cancer/non-cancer bodily fluid sample data and/or the cancer/non-cancer non-bodily fluid sample data in the training data set 410 according to one or more feature selection techniques. The training module 420 may train the ML module 430 by extracting a feature set from the training data set 410 that includes statistically significant features.

The training module 420 may extract a feature set from the training data set 410 in a variety of ways. The training module 420 may perform feature extraction multiple times, each time using a different feature-extraction technique. In an example, the feature sets generated using the different techniques may each be used to generate different machine learning-based classification models 440. For example, the feature set with the highest quality metrics may be selected for use in training. The training module 420 may use the feature set(s) to build one or more machine learning-based classification models 440A-440N that are configured to classify an origin as tumor or non-tumor for a new variant (e.g., with an unknown origin).

The training data set 410 may be analyzed to determine any dependencies, associations, and/or correlations between features and the experimental parameters in the training data set 410. The identified correlations may have the form of a list of features. The term "feature," as used herein, may refer to any characteristic of an item of data that may be used to determine whether the item of data falls within one or more specific categories. By way of example, the features described herein may comprise one or more of: frequency of observance of a genetic variant among samples of particular cancer type, including hematological malignancies; prevalence of variants in plasma, tumor tissue, or white blood cells; and/or minor allele frequency of a variant.

A feature selection technique may comprise one or more feature selection rules. The one or more feature selection rules may comprise a feature occurrence rule. The feature occurrence rule may comprise determining which features in the training data set 410 occur over a threshold number of times and identifying those features that satisfy the threshold as features.

A single feature selection rule may be applied to select features or multiple feature selection rules may be applied to select features. The feature selection rules may be applied in a cascading fashion, with the feature selection rules being applied in a specific order and applied to the results of the previous rule. For example, the feature occurrence rule may be applied to the training data set 410 to generate a first list of features. A final list of features may be analyzed according to additional feature selection techniques to determine one or more feature groups (e.g., groups of features that may be used to classify a variant as tumor origin or non-tumor origin). Any suitable computational technique may be used to identify the feature groups using any feature selection technique such as filter, wrapper, and/or embedded methods. One or more feature groups may be selected according to a filter method. Filter methods include, for example, Pearson's correlation, linear discriminant analysis, analysis of variance (ANOVA), chi-square, combinations thereof, and the like. The selection of features according to filter methods are independent of any machine learning algorithms. Instead, features may be selected on the basis of scores in various statistical tests for their correlation with the outcome variable.

As another example, one or more feature groups may be selected according to a wrapper method. A wrapper method may be configured to use a subset of features and train a machine learning model using the subset of features. Based on the inferences that drawn from a previous model, features may be added and/or deleted from the subset. Wrapper methods include, for example, forward feature selection, backward feature elimination, recursive feature elimination, combinations thereof, and the like. As an example, forward feature selection may be used to identify one or more feature groups. Forward feature selection is an iterative method that begins with no feature in the machine learning model. In each iteration, the feature which best improves the model is added until an addition of a new variable does not improve the performance of the machine learning model. As an example, backward elimination may be used to identify one or more feature groups. Backward elimination is an iterative method that begins with all features in the machine learning model. In each iteration, the least significant feature is removed until no improvement is observed on removal of features. Recursive feature elimination may be used to identify one or more feature groups. Recursive feature elimination is a greedy optimization algorithm which aims to find the best performing feature subset. Recursive feature elimination repeatedly creates models and keeps aside the best or the worst performing feature at each iteration. Recursive feature elimination constructs the next model with the features remaining until all the features are exhausted. Recursive feature elimination then ranks the features based on the order of their elimination.

As a further example, one or more feature groups may be selected according to an embedded method. Embedded methods combine the qualities of filter and wrapper methods. Embedded methods include, for example, Least Absolute Shrinkage and Selection Operator (LASSO) and ridge regression which implement penalization functions to reduce overfitting. For example, LASSO regression performs L1 regularization which adds a penalty equivalent to absolute value of the magnitude of coefficients and ridge regression performs L2 regularization which adds a penalty equivalent to square of the magnitude of coefficients.

After the training module 420 has generated a feature set(s), the training module 420 may generate a machine learning-based classification model 440 based on the feature set(s). A machine learning-based classification model may refer to a complex mathematical model for data classification that is generated using machine-learning techniques. In one example, the machine learning-based classification model 440 may include a map of support vectors that represent boundary features. By way of example, boundary features may be selected from, and/or represent the highest-ranked features in, a feature set.

The training module 420 may use the feature sets determined or extracted from the training data set 410 to build a machine learning-based classification model 440A-440N. In some examples, the machine learning-based classification models 440A-440N may be combined into a single machine learning-based classification model 440. Similarly, the ML module 430 may represent a single classifier containing a single or a plurality of machine learning-based classification models 440 and/or multiple classifiers containing a single or a plurality of machine learning-based classification models 440.

The features may be combined in a classification model trained using a machine learning approach such as discriminant analysis; decision tree; a nearest neighbor (NN) algorithm (e.g., k-NN models, replicator NN models, etc.); statistical algorithm (e.g., Bayesian networks, etc.); clustering algorithm (e.g., k-means, mean-shift, etc.); neural networks (e.g., reservoir networks, artificial neural networks, etc.); support vector machines (SVMs); logistic regression algorithms; linear regression algorithms; Markov models or chains; principal component analysis (PCA) (e.g., for linear models); multi-layer perceptron (MLP) ANNs (e.g., for non-linear models); replicating reservoir networks (e.g., for non-linear models, typically for time series); random forest classification; a combination thereof and/or the like. The resulting ML module 430 may comprise a decision rule or a mapping for each feature to determine tumor/non-tumor origin for a variant.

In an embodiment, the training module 420 may train the machine learning-based classification models 440 as a convolutional neural network (CNN). The CNN comprises at least one convolutional feature layer and three fully connected layers leading to a final classification layer (softmax). The final classification layer may finally be applied to combine the outputs of the fully connected layers using softmax functions as is known in the art.

The feature(s) and the ML module 430 may be used to predict the tumor/non-tumor origin of variants in the testing data set. In one example, the prediction result for each variant may include a confidence level that corresponds to a likelihood or a probability that a variant in the testing data set is associated with tumor origin or non-tumor origin. The confidence level may be a value between zero and one. In one example, when there are two statuses (e.g., tumor origin and non-tumor origin), the confidence level may correspond to a value p, which refers to a likelihood that a particular variant belongs to the first status (e.g., tumor origin). In this case, the value 1-$p$ may refer to a likelihood that the particular variant belongs to the second status (e.g., non-tumor origin). In general, multiple confidence levels may be provided for each variant in the testing data set and for each feature when there are more than two statuses. A top performing feature may be determined by comparing the result obtained for each test variant with the known tumor/non-tumor origin for each test variant. In general, the top performing feature will have results that closely match the known tumor/non-tumor origin statuses. The top performing feature(s) may be used to predict/classify the tumor/non-tumor origin status of a given variant.

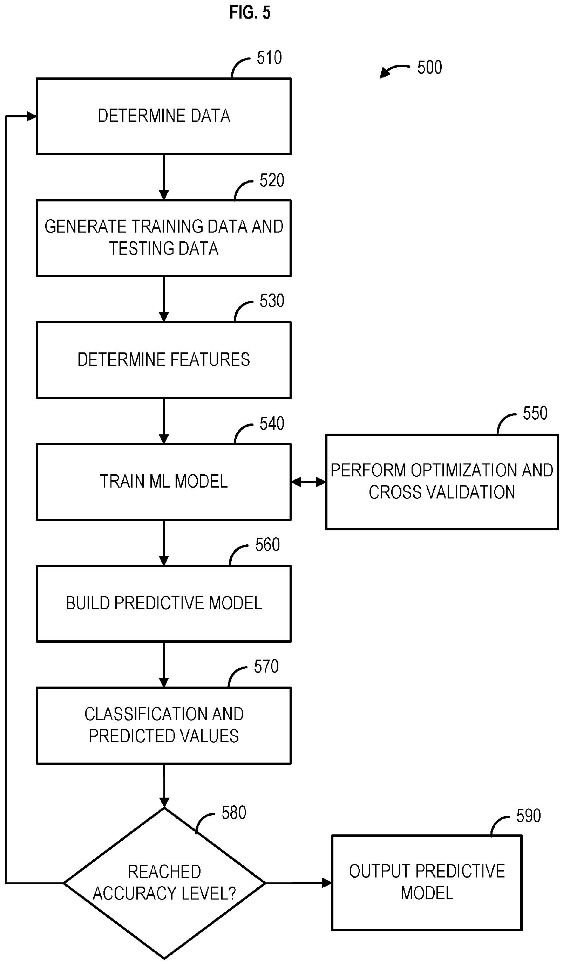

FIG. 5 is a flowchart illustrating an example training method 500 for generating the ML module 430 using the training module 420. The training module 420 can implement supervised, unsupervised, and/or semi-supervised (e.g.,

reinforcement based) machine learning-based classification models 440. The method 500 illustrated in FIG. 5 is an example of a supervised learning method; variations of this example of training method are discussed below, however, other training methods can be analogously implemented to train unsupervised and/or semi-supervised machine learning models.

The training method 500 may determine (e.g., access, receive, retrieve, etc.) data at step 510. The data may comprise cancer/non-cancer (e.g., tumor/non-tumor) bodily fluid sample data and cancer/non-cancer (e.g., tumor/non-tumor) non-bodily fluid (e.g., tissue) sample data. The data may comprise one or more variants, each variant having an assigned tumor or non-tumor origin status.

The training method 500 may generate, at step 520, a training data set and a testing data set. The training data set and the testing data set may be generated by randomly assigning data to either the training data set or the testing data set. In some implementations, the assignment of computation parameters and associated experimental parameters as training or testing data may not be completely random. As an example, a majority of the computation parameters and associated experimental parameters may be used to generate the training data set. For example, 75% of the computation parameters and associated experimental parameters may be used to generate the training data set and 25% may be used to generate the testing data set. In another example, 80% of the computation parameters and associated experimental parameters may be used to generate the training data set and 20% may be used to generate the testing data set.

The training method 500 may determine (e.g., extract, select, etc.), at step 530, one or more features that can be used by, for example, a classifier to differentiate among different classification of tumor vs. non-tumor status. As an example, the training method 500 may determine a set of features from the cancer/non-cancer bodily fluid sample data and cancer/non-cancer non-bodily fluid sample data. In a further example, a set of features may be determined from data that is different than the the cancer/non-cancer bodily fluid sample data and cancer/non-cancer non-bodily fluid sample data in either the training data set or the testing data set. Such other data may be used to determine an initial set of features, which may be further reduced using the training data set.

The training method 500 may train one or more machine learning models using the one or more features at step 540. In one example, the machine learning models may be trained using supervised learning. In another example, other machine learning techniques may be employed, including unsupervised learning and semi-supervised. The machine learning models trained at 540 may be selected based on different criteria depending on the problem to be solved and/or data available in the training data set. For example, machine learning classifiers can suffer from different degrees of bias. Accordingly, more than one machine learning model can be trained at 540, optimized, improved, and cross-validated at step 550.

The training method 500 may select one or more machine learning models to build a predictive model at 560. The predictive model may be evaluated using the testing data set. The predictive model may analyze the testing data set and generate predicted tumor/non-tumor origin statuses at step 570. Predicted tumor/non-tumor origin may be evaluated at step 580 to determine whether such values have achieved a desired accuracy level. Performance of the predictive model may be evaluated in a number of ways based on a number of true positives, false positives, true negatives, and/or false negatives classifications of the plurality of data points indicated by the predictive model.

For example, the false positives of the predictive model may refer to a number of times the predictive model incorrectly classified a variant as tumor origin that was in reality non-tumor origin. Conversely, the false negatives of the predictive model may refer to a number of times the machine learning model classified a variant as non-tumor origin when, in fact, the variant was tumor origin. True negatives and true positives may refer to a number of times the predictive model correctly classified one or more variants. Related to these measurements are the concepts of recall and precision. Generally, recall refers to a ratio of true positives to a sum of true positives and false negatives, which quantifies a sensitivity of the predictive model. Similarly, precision refers to a ratio of true positives a sum of true and false positives. When such a desired accuracy level is reached, the training phase ends and the predictive model (e.g., the ML module 430) may be output at step 590; when the desired accuracy level is not reached, however, then a subsequent iteration of the training method 500 may be performed starting at step 510 with variations such as, for example, considering a larger collection of data.

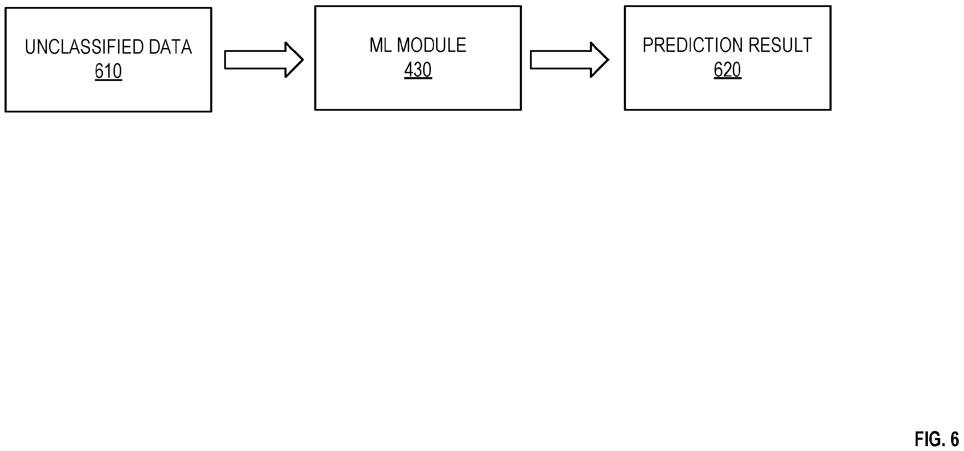

FIG. 6 is an illustration of an exemplary process flow for using a machine learning-based classifier to classify a variant as tumor origin or non-tumor origin. As illustrated in FIG. 6, an unclassified variant 610 may be provided as input to the ML module 430. The ML module 430 may process the unclassified variant 610 using a machine learning-based classifier(s) to arrive at a prediction result 620. The prediction result 620 may identify one or more characteristics of the unclassified variant 610. For example, the classification result 620 may identify the origin status of the unclassified variant 610 (e.g., whether the variant is tumor origin or non-tumor origin). Thus, in an embodiment, dislosed is a method implemented using a network-based computer system comprising one or more processors, a network interface, and one or more memories, the method comprising retrieving, by the computer system, genetic information and additional information of a plurality of tumor and non-tumor bodily fluid samples and a plurality of tumor and non-tumor non-bodily fluid (e.g., tissue) samples from the one or more memories, wherein the additional information comprises a tumor origin or non-tumor origin status; and training, by the one or more processors, a machine-learning model by fitting one or more models to the genetic information and additional information, wherein each of the one or more models is configured to receive as input genetic information of an individual, and provide as output a prediction of the individual having or developing a tumor.

Systems and Computer Readable Media

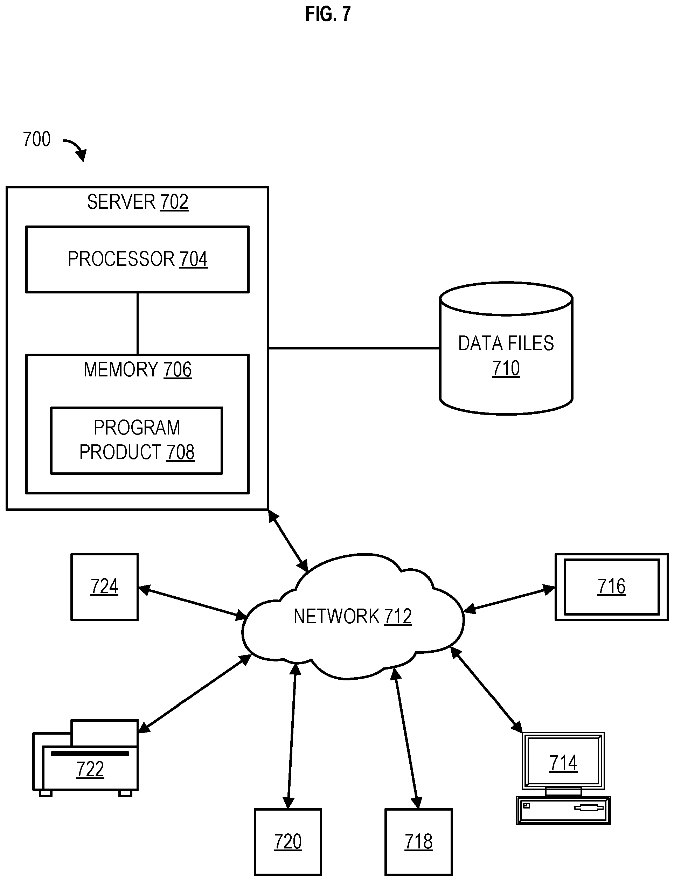

The present disclosure also provides various systems, bioinformatics pipelines, and computer program products or machine readable media. In some embodiments, for example, the methods described herein are optionally performed or facilitated at least in part using systems, distributed computing hardware and applications (e.g., cloud computing services), electronic communication networks, communication interfaces, computer program products, machine readable media, electronic storage media, software (e.g., machine-executable code or logic instructions) and/or the like. To illustrate, FIG. 7 provides a schematic diagram of an exemplary system suitable for use with implementing at least aspects of the methods disclosed in this application. As shown, system 700 includes at least one controller or computer, e.g., server 702 (e.g., a search engine server), which includes processor 704 and memory, storage device, or memory component 706, and one or more other communication devices 714 and 716 (e.g., client-side computer terminals, telephones, tablets, laptops, other mobile devices, etc.) positioned remote from and in communication with the remote server 702, through electronic communication network 712, such as the Internet or other internetwork. Communication devices 714 and 716 typically include an electronic display (e.g., an internet enabled computer or the like) in communication with, e.g., server 702 computer over network 712 in which the electronic display comprises a user interface (e.g., a graphical user interface (GUI), a web-based user interface, and/or the like) for displaying results upon implementing the methods described herein. In certain embodiments, communication networks also encompass the physical transfer of data from one location to another, for example, using a hard drive, thumb drive, or other data storage mechanism. System 700 also includes program product 708 stored on a computer or machine readable medium, such as, for example, one or more of various types of memory, such as memory 706 of server 702, that is readable by the server 702, to facilitate, for example, a guided search application or other executable by one or more other communication devices, such as 714 (schematically shown as a desktop or personal computer) and 716 (schematically shown as a tablet computer). In some embodiments, system 700 optionally also includes at least one database server, such as, for example, server 710 associated with an online website having data stored thereon (e.g., nucleic acid variant lists, indexed therapies, etc.) searchable either directly or through search engine server 702. System 700 optionally also includes one or more other servers positioned remotely from server 702, each of which are optionally associated with one or more database servers 710 located remotely or located local to each of the other servers. The other servers can beneficially provide service to geographically remote users and enhance geographically distributed operations.

As understood by those of ordinary skill in the art, memory 706 of the server 702 optionally includes volatile and/or nonvolatile memory including, for example, RAM, ROM, and magnetic or optical disks, among others. It is also understood by those of ordinary skill in the art that although illustrated as a single server, the illustrated configuration of server 702 is given only by way of example and that other types of servers or computers configured according to various other methodologies or architectures can also be used. Server 702 shown schematically in FIG. 7, represents a server or server cluster or server farm and is not limited to any individual physical server. The server site may be deployed as a server farm or server cluster managed by a server hosting provider. The number of servers and their architecture and configuration may be increased based on usage, demand and capacity requirements for the system 700. As also understood by those of ordinary skill in the art, other user communication devices 714 and 716 in these embodiments, for example, can be a laptop, desktop, tablet, personal digital assistant (PDA), cell phone, server, or other types of computers. As known and understood by those of ordinary skill in the art, network 712 can include an internet, intranet, a telecommunication network, an extranet, or world wide web of a plurality of computers/servers in communication with one or more other computers through a communication network, and/or portions of a local or other area network.

As further understood by those of ordinary skill in the art, exemplary program product or machine readable medium 708 is optionally in the form of microcode, programs, cloud computing format, routines, and/or symbolic languages that provide one or more sets of ordered operations that control the functioning of the hardware and direct its operation. Program product 708, according to an exemplary embodiment, also need not reside in its entirety in volatile memory, but can be selectively loaded, as necessary, according to various methodologies as known and understood by those of ordinary skill in the art.

As further understood by those of ordinary skill in the art, the term "computer-readable medium" or "machine-readable medium" refers to any medium that participates in providing instructions to a processor for execution. To illustrate, the term "computer-readable medium" or "machine-readable medium" encompasses distribution media, cloud computing formats, intermediate storage media, execution memory of a computer, and any other medium or device capable of storing program product 708 implementing the functionality or processes of various embodiments of the present disclosure, for example, for reading by a computer. A "computer-readable medium" or "machine-readable medium" may take many forms, including but not limited to, non-volatile media, volatile media, and transmission media. Non-volatile media includes, for example, optical or magnetic disks. Volatile media includes dynamic memory, such as the main memory of a given system. Transmission media includes coaxial cables, copper wire and fiber optics, including the wires that comprise a bus. Transmission media can also take the form of acoustic or light waves, such as those generated during radio wave and infrared data communications, among others. Exemplary forms of computer-readable media include a floppy disk, a flexible disk, hard disk, magnetic tape, a flash drive, or any other magnetic medium, a CD-ROM, any other optical medium, punch cards, paper tape, any other physical medium with patterns of holes, a RAM, a PROM, and EPROM, a FLASH-EPROM, any other memory chip or cartridge, a carrier wave, or any other medium from which a computer can read.

Program product 708 is optionally copied from the computer-readable medium to a hard disk or a similar intermediate storage medium. When program product 708, or portions thereof, are to be run, it is optionally loaded from their distribution medium, their intermediate storage medium, or the like into the execution memory of one or more computers, configuring the computer(s) to act in accordance with the functionality or method of various embodiments. All such operations are well known to those of ordinary skill in the art of, for example, computer systems.

To further illustrate, in certain embodiments, this application provides systems that include one or more processors, and one or more memory components in communication with the processor. The memory component typically includes one or more instructions that, when executed, cause the processor to provide information that causes at least one nucleic acid variant list, variant classification call report or result, selected therapies, and/or the like to be displayed (e.g., via communication devices 714, 716, or the like) and/or receive information from other system components and/or from a system user (e.g., via communication devices 714, 716, or the like).

In some embodiments, program product 708 includes non-transitory computer-executable instructions which, when executed by electronic processor 704 perform at least: (i) generating a tumor variant dataset that includes a population of reference tumor-related genetic variants in which the tumor variant dataset includes frequency of observance data among reference samples that includes reference bodily fluid samples and reference non-bodily fluid samples for tumor-related genetic variants in the population of reference tumor-related genetic variants and in which the reference samples are obtained from a single reference subject and/or from different reference subjects having an identical cancer type, (ii) determining ratios of the frequency of observance data between the reference samples for tumor-related genetic variants in the population of reference tumor-related genetic variants to produce a relative prevalence dataset, (iii) generating a set of probabilities of non-tumor origin from the relative prevalence dataset, and (iv) using the set of probabilities of non-tumor origin to differentiate nucleic acid variants detected in the cfNA sample obtained from the test subject as being tumor origin nucleic acid variants or non-tumor origin nucleic acid variants.

System 700 also typically includes additional system components that are configured to perform various aspects of the methods described herein. In some of these embodiments, one or more of these additional system components are positioned remote from and in communication with the remote server 702 through electronic communication network 712, whereas in other embodiments, one or more of these additional system components are positioned local, and in communication with server 702 (i.e., in the absence of electronic communication network 712) or directly with, for example, desktop computer 714.

In some embodiments, for example, additional system components include sample preparation component 718 is operably connected (directly or indirectly (e.g., via electronic communication network 712)) to controller 702. Sample preparation component 718 is configured to prepare the nucleic acids in samples (e.g., prepare libraries of nucleic acids) to be amplified and/or sequenced by a nucleic acid amplification component (e.g., a thermal cycler, etc.) and/or a nucleic acid sequencer. In certain of these embodiments, sample preparation component 718 is configured to isolate nucleic acids from other components in a sample, to attach one or adapters comprising barcodes to nucleic acids as described herein, selectively enrich one or more regions from a genome or transcriptome prior to sequencing, and/or the like.

In certain embodiments, system 700 also includes nucleic acid amplification component 720 (e.g., a thermal cycler, etc.) operably connected (directly or indirectly (e.g., via electronic communication network 712)) to controller 702. Nucleic acid amplification component 720 is configured to amplify nucleic acids in samples from subjects. For example, nucleic acid amplification component 720 is optionally configured to amplify selectively enriched regions from a genome or transcriptome in the samples as described herein.

System 700 also typically includes at least one nucleic acid sequencer 722 operably connected (directly or indirectly (e.g., via electronic communication network 712)) to controller 702. Nucleic acid sequencer 722 is configured to provide the sequence information from nucleic acids (e.g., amplified nucleic acids) in samples from subjects. Essentially any type of nucleic acid sequencer can be adapted for use in these systems. For example, nucleic acid sequencer 722 is optionally configured to perform pyrosequencing, single-molecule sequencing, nanopore sequencing, semiconductor sequencing, sequencing-by-ligation, sequencing-by-hybridization, or other techniques on the nucleic acids to generate sequencing reads. Optionally, nucleic acid sequencer 722 is configured to group sequence reads into families of sequence reads, each family comprising sequence reads generated from a nucleic acid in a given sample. In some embodiments, nucleic acid sequencer 722 uses a clonal single molecule array derived from the sequencing library to generate the sequencing reads. In certain embodiments, nucleic acid sequencer 722 includes at least one chip having an array of microwells for sequencing a sequencing library to generate sequencing reads.

To facilitate complete or partial system automation, system 700 typically also includes material transfer component 724 operably connected (directly or indirectly (e.g., via electronic communication network 712)) to controller 702. Material transfer component 724 is configured to transfer one or more materials (e.g., nucleic acid samples, amplicons, reagents, and/or the like) to and/or from nucleic acid sequencer 722, sample preparation component 718, and nucleic acid amplification component 720.

Additional details relating to computer systems and networks, databases, and computer program products are also provided in, for example, Peterson, Computer Networks: A Systems Approach, Morgan Kaufmann, 5th Ed. (2011), Kurose, Computer Networking: A Top-Down Approach, Pearson, 7th Ed. (2016), Elmasri, Fundamentals of Database Systems, Addison Wesley, 6th Ed. (2010), Coronel, Database Systems: Design, Implementation, & Management, Cengage Learning, 11th Ed. (2014), Tucker, Programming Languages, McGraw-Hill Science/Engineering/Math, 2nd Ed. (2006), and Rhoton, Cloud Computing Architected: Solution Design Handbook, Recursive Press (2011).

Sample Collection and Preparation

A sample may be any biological sample isolated from a subject. Samples can include bodily fluid or bodily tissues (e.g., known or suspected solid tumors). Samples can include whole blood, platelets, serum, plasma, stool, red blood cells, white blood cells or leucocytes, endothelial cells, tissue biopsies, cerebrospinal fluid synovial fluid, lymphatic fluid, ascites fluid, interstitial or extracellular fluid, the fluid in spaces between cells, including gingival crevicular fluid, bone marrow, pleural effusions, cerebrospinal fluid, saliva, mucous, sputum, semen, sweat, urine. Samples are preferably bodily fluids, particularly blood and fractions thereof, and urine. Such samples include nucleic acids shed from tumors. The nucleic acids can include DNA and RNA and can be in double- and/or single-stranded forms. A sample can be in the form originally isolated from a subject or can have been subjected to further processing to remove or add components, such as cells, enrich for one component relative to another, or convert one form of nucleic acid to another, such as RNA to DNA or single-stranded nucleic acids to double-stranded. Thus, for example, a bodily fluid for analysis is plasma or serum containing cell-free nucleic acids, e.g., cell-free DNA (cfDNA).

In certain embodiments, the polynucleotides can be enriched prior to sequencing. Enrichment can be performed for specific target regions ("target sequences") or nonspecifically. In some embodiments, targeted regions of interest may be enriched with capture probes ("baits") selected for one or more bait set panels using a differential tiling and capture scheme. A differential tiling and capture scheme uses bait sets of different relative concentrations to differentially tile (e.g., at different "resolutions") across genomic regions associated with baits, subject to a set of constraints (e.g., sequencer constraints such as sequencing load, utility of each bait, etc.), and capture them at a desired level for downstream sequencing. These targeted genomic regions of interest may include regions of a subject's genome or transcriptome. In some embodiments, biotin-labeled beads with probes to one or more regions of interest can be used to capture target sequences, optionally followed by amplification of those regions, to enrich for the regions of interest.

Sequence capture typically involves the use of oligonucleotide probes that hybridize to the target sequence. A probe set strategy can involve tiling the probes across a region of interest. Such probes can be, e.g., about 60 to 130 bases long. The set can have a depth of about 2x, 3x, 4x, 5x, 6x, 8x, 9x, 10x, 15x, 30x, 50x, or more. The effectiveness of sequence capture depends, in part, on the length of the sequence in the target molecule that is complementary (or nearly complementary) to the sequence of the probe.

In some embodiments, the methods of the disclosure comprise selectively enriching regions from the subject's genome or transcriptome prior to sequencing. In other embodiments, the methods of the disclosure comprise non-selectively enriching regions from the subject's genome or transcriptome prior to sequencing.

In certain embodiments, sample index sequences are introduced to the polynucleotides after enrichment. The sample index sequences may be introduced through PCR or ligated to the polynucleotides, optionally as part of adapters.

The volume of bodily fluid can depend on the desired read depth for sequenced regions. Exemplary volumes are 0.4-40 ml, 5-20 ml, 10-20 ml. For example, the volume can be 0.5 ml, 1 ml, 5 ml, 10 ml, 20 ml, 30 ml, or 40 ml. A volume of sampled bodily fluid may be 5 to 20 ml.

The sample can comprise various amounts of nucleic acid that contains genome equivalents. For example, a sample of about 30 ng DNA can contain about 10,000 ($10^4$) haploid human genome equivalents and, in the case of cfDNA, about 200 billion ($2 \times 10^{11}$) individual polynucleotide molecules. Similarly, a sample of about 100 ng of DNA can contain about 30,000 haploid human genome equivalents and, in the case of cfDNA, about 600 billion individual molecules.

A sample can comprise nucleic acids from different sources, e.g., from cells and cell free. A sample can comprise nucleic acids carrying mutations. For example, a sample can comprise DNA carrying germline mutations and/or somatic mutations. A sample can comprise DNA carrying cancer-associated mutations (e.g., cancer-associated somatic mutations).

Exemplary amounts of cell free nucleic acids in a sample before amplification range from about 1 fg to about 1 μg, e.g., 1 pg to 200 ng, 1 ng to 100 ng, 10 ng to 1000 ng. For example, the amount can be up to about 600 ng, up to about 500 ng, up to about 400 ng, up to about 300 ng, up to about 200 ng, up to about 100 ng, up to about 50 ng, or up to about 20 ng of cell-free nucleic acid molecules. The amount can be at least 1 fg, at least 10 fg, at least 100 fg, at least 1 pg, at least 10 pg, at least 100 pg, at least 1 ng, at least 10 ng, at least 100 ng, at least 150 ng, or at least 200 ng of cell-free nucleic acid molecules. The amount can be up to 1 femtogram (fg), 10 fg, 100 fg, 1 picogram (pg), 10 pg, 100 pg, 1 ng, 10 ng, 100 ng, 150 ng, or 200 ng of cell-free nucleic acid molecules. The method can comprise obtaining 1 femtogram (fg) to 200 ng.

Cell-free nucleic acids have an exemplary size distribution of about 100-500 nucleotides, with molecules of 110 to about 230 nucleotides representing about 90% of molecules, with a mode of about 168 nucleotides in humans and a second minor peak in a range between 240 to 430 nucleotides. Cell-free nucleic acids can be about 160 to about 180 nucleotides, or about 320 to about 360 nucleotides, or about 430 to about 480 nucleotides.

Cell-free nucleic acids can be isolated from bodily fluids through a partitioning step in which cell-free nucleic acids, as found in solution, are separated from intact cells and other non-soluble components of the bodily fluid. Partitioning may include techniques such as centrifugation or filtration. Alternatively, cells in bodily fluids can be lysed and cell-free and cellular nucleic acids processed together. Generally, after addition of buffers and wash steps, cell-free nucleic acids can be precipitated with an alcohol. Further clean up steps may be used such as silica based columns to remove contaminants or salts. Non-specific bulk carrier nucleic acids, for example, may be added throughout the reaction to optimize certain aspects of the procedure such as yield.

After such processing, samples can include various forms of nucleic acid including double-stranded DNA, single stranded DNA and single stranded RNA. Optionally, single stranded DNA and RNA can be converted to double-stranded forms so they are included in subsequent processing and analysis steps.

Amplification

Sample nucleic acids flanked by adapters can be amplified by PCR and other amplification methods typically primed from primers binding to primer binding sites in adapters flanking a DNA molecule to be amplified. Amplification methods can involve cycles of extension, denaturation and annealing resulting from thermocycling or can be isothermal as in transcription mediated amplification. Other amplification methods include the ligase chain reaction, strand displacement amplification, nucleic acid sequence-based amplification, and self-sustained sequence-based replication.

One or more amplifications can be applied to introduce barcodes to a nucleic acid molecule using conventional nucleic acid amplification methods. The amplification can be conducted in one or more reaction mixtures. Molecule tags and sample indexes/tags can be introduced simultaneously, or in any sequential order. Molecule tags and sample indexes/tags can be introduced prior to and/or after sequence capturing. In some cases, only the molecule tags are introduced prior to probe capturing while the sample indexes/tags are introduced after sequence capturing. In some cases, both the molecule tags and the sample indexes/tags are introduced prior to probe capturing. In some cases, the sample indexes/tags are introduced after sequence capturing. Usually, sequence capturing involves introducing a single-stranded nucleic acid molecule complementary to a targeted sequence, e.g., a coding sequence of a genomic region and mutation of such region is associated with a cancer type. Typically, the amplifications generate a plurality of non-uniquely or uniquely tagged nucleic acid amplicons with molecule tags and sample indexes/tags at a size ranging from 200 nt to 700 nt, 250 nt to 350 nt, or 320 nt to 550 nt. In some embodiments, the amplicons have a size of about 300 nt. In some embodiments, the amplicons have a size of about 500 nt.

Barcodes

Barcodes can be incorporated into or otherwise joined to adapters by chemical synthesis, ligation, overlap extension PCR among other methods. Generally, assignment of unique or non-unique barcodes in reactions follows methods and systems described by US patent applications 20010053519, 20110160078, and U.S. Pat. No. 6,582,908 and U.S. Pat. No. 7,537,898 and US 9,598,731.

Tags can be linked to sample nucleic acids randomly or non-randomly. In some cases, they are introduced at an expected ratio of identifiers (i.e., a combination of barcodes) to microwells. The collection of barcodes can be unique, e.g., all the barcodes have a different nucleotide sequence. The collection of barcodes can be non-unique, i.e., some of the barcodes have the same nucleotide sequence, and some of the barcodes have different nucleotide sequence. For example, the identifiers may be loaded so that more than 1, 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 identifiers are loaded per genome sample. In some cases, the identifiers may be loaded so that less than 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 identifiers are loaded per genome sample. In some cases, the average number of identifiers loaded per sample genome is less than, or greater than, about 1, 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 identifiers per genome sample.

A preferred format uses 20-50 different tags, ligated to both ends of a target molecule creating 20-50 × 20-50 tags, i.e., 400-2500 tag combinations. Such numbers of tags are sufficient that different molecules having the same start and stop points have a high probability (e.g., at least 94%, 99.5%, 99.99%, 99.999%) of receiving different combinations of tags.

In some cases, identifiers may be predetermined or random or semi-random sequence oligonucleotides. In other cases, a plurality of barcodes may be used such that barcodes are not necessarily unique to one another in the plurality. In this example, barcodes may be attached (e.g., by ligation or PCR amplification) to individual molecules such that the combination of the barcode and the sequence it may be attached to creates a unique sequence that may be individually tracked. As described herein, detection of non-uniquely tagged barcodes in combination with beginning (start) and/or end (stop) genomic coordinates of a given sequenced sample molecule (i.e., excluding sequence information obtained from the barcodes, adaptors, and the like)) may allow assignment of a unique identity to a particular molecule. The length, or number of base pairs, of an individual sequenced sample molecule (i.e., exclusive of sequence information corresponding to barcodes, adaptors, and the like) may also be used to assign a unique identity to such a molecule. As described herein, fragments from a single strand of nucleic acid having been assigned a unique identity, may thereby permit subsequent identification of fragments from the parent strand, and/or a complementary strand.

Sequencing Pipeline

Sample nucleic acids flanked by adapters with or without prior amplification can be subject to sequencing, such as by one or more sequencing devices 107. Sequencing methods include, for example, Sanger sequencing, high-throughput sequencing, pyrosequencing, sequencing-by-synthesis, single-molecule sequencing, nanopore sequencing, semiconductor sequencing, sequencing-by-ligation, sequencing-by-hybridization, RNA-Seq (Illumina), Digital Gene Expression (Helicos), Next generation sequencing, Single Molecule Sequencing by Synthesis (SMSS) (Helicos), massively-parallel sequencing, Clonal Single Molecule Array (Solexa), shotgun sequencing, Ion Torrent, Oxford Nanopore, Roche Genia, Maxim-Gilbert sequencing, primer walking, sequencing using PacBio, SOLiD, Ion Torrent, or Nanopore platforms. Sequencing reactions can be performed in a variety of sample processing units, which may be multiple lanes, multiple channels, multiple wells, or other means of processing multiple sample sets substantially simultaneously. Sample processing unit can also include multiple sample chambers to enable processing of multiple runs simultaneously.

The sequencing reactions can be performed on one or more fragments types known to contain markers of cancer of other disease. The sequencing reactions can also be performed on any nucleic acid fragments present in the sample. The sequence reactions may provide for sequencing at least 5%, 10%, 15%, 20%, 25%, 30%, 40%, 50%, 60%, 70%, 80%, 90%, 95%, 99%, 99.9% or 100% of a given genome. In other cases, the sequence reactions may provide for sequencing less than 5%, 10%, 15%, 20%, 25%, 30%, 40%, 50%, 60%, 70%, 80%, 90%, 95%, 99%, 99.9% or 100% of a given genome.

Simultaneous sequencing reactions may be performed using multiplex sequencing. In some cases, cell free polynucleotides may be sequenced with at least 1000, 2000, 3000, 4000, 5000, 6000, 7000, 8000, 9000, 10000, 50000, 100,000 sequencing reactions. In other cases, cell free polynucleotides may be sequenced with less than 1000, 2000, 3000, 4000, 5000, 6000, 7000, 8000, 9000, 10000, 50000, 100,000 sequencing reactions. Sequencing reactions may be performed sequentially or simultaneously. Subsequent data analysis may be performed on all or part of the sequencing reactions. In some cases, data analysis may be performed on at least 1000, 2000, 3000, 4000, 5000, 6000, 7000, 8000, 9000, 10000, 50000, 100,000 sequencing reactions. In other cases, data analysis may be performed on less than 1000, 2000, 3000, 4000, 5000, 6000, 7000, 8000, 9000, 10000, 50000, 100,000 sequencing reactions. An exemplary read depth is 1000-50000 reads per locus (base).

Sequence Analysis Pipeline

Nucleotide variations in sequenced nucleic acids can be determined by comparing sequenced nucleic acids with a reference sequence. The reference sequence is often a known sequence, e.g., a known whole or partial genome sequence from an object, whole genome sequence of a human object. The reference sequence can be hG19. The sequenced nucleic acids can represent sequences determined directly for a nucleic acid in a sample, or a consensus of sequences of amplification products of such a nucleic acid, as described above. A comparison can be performed at one or more designated positions on a reference sequence. A subset of sequenced nucleic acids can be identified including a position corresponding with a designated position of the reference sequence when the respective sequences are maximally aligned. Within such a subset it can be determined which, if any, sequenced nucleic acids include a nucleotide variation at the designated position, and optionally which if any, include a reference nucleotide (i.e., same as in the reference sequence). If the number of sequenced nucleic acids in the subset including a nucleotide variant exceeds a threshold, then a variant nucleotide can be called at the designated position. The threshold can be a simple number, such as at least 1, 2, 3, 4, 5, 6, 7, 8, 9, or 10 sequenced nucleic acid within the subset including the nucleotide variant or it can be a ratio, such as a least 0.5, 1, 2, 3, 4, 5, 10, 15, or 20 of sequenced nucleic acids within the subset include the nucleotide variant, among other possibilities. The comparison can be repeated for any designated position of interest in the reference sequence. Sometimes a comparison can be performed for designated positions occupying at least 20, 100, 200, or 300 contiguous positions on a reference sequence, e.g., 20-500, or 50-300 contiguous positions.

The present methods can also be used to diagnose the presence or absence of conditions, particularly cancer, in a subject, to characterize conditions (e.g., staging cancer or determining heterogeneity of a cancer), monitor response to treatment of a condition, effect prognosis risk of developing a condition or subsequent course of a condition.

Various cancers may be detected using the present methods. Cancer cells, as most cells, can be characterized by a rate of turnover, in which old cells die and replaced by newer cells. Generally dead cells, in contact with vasculature in a given subject, may release DNA or fragments of DNA into the blood stream. This is also true of cancer cells during various stages of the disease. Cancer cells may also be characterized, dependent on the stage of the disease, by various genetic aberrations such as copy number variation as well as rare mutations. This phenomenon may be used to detect the presence or absence of cancer in individuals using the methods and systems described herein.

The types and number of cancers that may be detected may include blood cancers, brain cancers, lung cancers, skin cancers, nose cancers, throat cancers, liver cancers, bone cancers, lymphomas, pancreatic cancers, skin cancers, bowel cancers, rectal cancers, thyroid cancers, bladder cancers, kidney cancers, mouth cancers, stomach cancers, solid state tumors, heterogeneous tumors, homogenous tumors and the like.

Cancers can be detected from genetic variations including mutations, rare mutations, indels, copy number variations, transversions, translocations, inversion, deletions, aneuploidy, partial aneuploidy, polyploidy, chromosomal instability, chromosomal structure alterations, gene fusions, chromosome fusions, gene truncations, gene amplification, gene duplications, chromosomal lesions, DNA lesions, abnormal changes in nucleic acid chemical modifications, abnormal changes in epigenetic patterns.

Genetic data can also be used for characterizing a specific form of cancer. Cancers are often heterogeneous in both composition and staging. Genetic profile data may allow characterization of specific sub-types of cancer that may be important in the diagnosis or treatment of that specific sub-type. This information may also provide a subject or practitioner clues regarding the prognosis of a specific type of cancer and allow either a subject or practitioner to adapt treatment options in accord with the progress of the disease. Some cancers progress, becoming more aggressive and genetically unstable. Other cancers may remain benign, inactive or dormant. The system and methods of this disclosure may be useful in determining disease progression.

The present analysis is also useful in determining the efficacy of a particular treatment option. Successful treatment options may increase the amount of copy number variation or rare mutations detected in a subject's blood if the treatment is successful as more cancers may die and shed DNA. In other examples, this may not occur. In another example, perhaps certain treatment options may be correlated with genetic profiles of cancers over time. This correlation may be useful in selecting a therapy. Additionally, if a cancer is observed to be in remission after treatment, the present methods can be used to monitor residual disease or recurrence of disease.

The present methods can also be used for detecting genetic variations in conditions other than cancer. Immune cells, such as B cells, may undergo rapid clonal expansion upon the presence certain diseases. Clonal expansions may be monitored using copy number variation detection and certain immune states may be monitored. In this example, copy number variation analysis may be performed over time to produce a profile of how a particular disease may be progressing. Copy number variation or even rare mutation detection may be used to determine how a population of pathogens are changing during the course of infection. This may be particularly important during chronic infections, such as HIV/AIDs or Hepatitis infections, whereby viruses may change life cycle state and/or mutate into more virulent forms during the course of infection. The present methods may be used to determine or profile rejection activities of the host body, as immune cells attempt to destroy transplanted tissue to monitor the status of transplanted tissue as well as altering the course of treatment or prevention of rejection.

Further, the methods of the disclosure may be used to characterize the heterogeneity of an abnormal condition in a subject, the method comprising generating a genetic profile of extracellular polynucleotides in the subject, wherein the genetic profile comprises a plurality of data resulting from copy number variation and rare mutation analyses. In some cases, including but not limited to cancer, a disease may be heterogeneous. Disease cells may not be identical. In the example of cancer, some tumors are known to comprise different types of tumor cells, some cells in different stages of the cancer. In other examples, heterogeneity may comprise multiple foci of disease. Again, in the example of cancer, there may be multiple tumor foci, perhaps where one or more foci are the result of metastases that have spread from a primary site.

The present methods can be used to generate or profile, fingerprint or set of data that is a summation of genetic information derived from different cells in a heterogeneous disease. This set of data may comprise copy number variation and rare mutation analyses alone or in combination.

The present methods can be used to diagnose, prognose, monitor or observe cancers or other diseases of fetal origin. That is, these methodologies may be employed in a pregnant subject to diagnose, prognose, monitor or observe cancers or other diseases in an unborn subject whose DNA and other polynucleotides may co-circulate with maternal molecules.

Exemplary Precision Treatments and Applications

The precision diagnostics provided by the computer system 700 may result in precision treatment plans, which may be identified by the computer system 700 (and/or curated by health professionals). For example, in lung cancer and other diseases, a goal may be to ensure that no superior treatment options exist, given presence of a given variant. For example, EGFR (L858R, exon 19 deletion), BRAF V600E, ALK, and ROS1 fusions may be treated with targeted therapies that may be more suitable than platinum- and chemo-therapies. Although these are examples of the primary drivers, other targetable drivers exist, such as MET exon 14 skipping. In another example, for colon cancer, the goal may be to avoid non-effective treatments. Chemotherapy with FOLFIRI or Chemotherapy with irinotecan regimens maybe supplemented with Cetuximab or Panitumumab if KRAS or NRAS is wildtype. Thus, confidence in whether KRAS and NRAS are wildtype will increase confidence that adding Cetuximab or Panitumumab is the correct treatment option and no further testing may be required. The biological explanation for this is that Cetuximab or Panitumumab Target EGFR and inhibit its activity. RAS (K/NRAS) is downstream of EGFR, so if RAS is activated, inhibiting EGFR will have minimal or no impact, so the Cetuximab or Panitumumab treatment will be administered inappropriately.

The variant analyzed by the methods and systems of the present disclosure may be a loss of function variant (such as ATM). For example, DNA damage repair (DDR) is a cellular process that functions to maintain genomic integrity or stability. Defects or deficiencies in a given DDR mechanism can lead to tumorigenesis or other diseases and can be used to identify test subjects or patients that may benefit from a given targeted therapy. Homologous recombination repair deficiency (HRD), as an example, is a cellular phenotype that may make patients candidates for the administration of therapeutic agents, such as poly ADP ribose polymerase (PARP) inhibitors. In certain embodiments, a therapy may be administered to a subject that comprises at least one PARP inhibitor, wherein the variant has been deteremined to be of tumor or non-tumor origin using the methods and systems described herein. In certain embodiments, the PARP inhibitor may include OLAPARIB, TALAZOPARIB, RUCAPARIB, NIRAPARIB (trade name ZEJULA), among others. In some embodiments, the therapies comprise at least one base excision repair (BER) inhibitor. For example, OLAPARIB may inhibit BER. In certain embodiments, administration of a therapy to a subject may be discontinued based on the determination that the subject has a variant of tumor or non-tumor origin using the methods and systems described herein.

Non-tumor variants could impact determination of a tumor mutation burden (TMB) score, which will result in an artificially high score if not removed or filtered from the TMB determination. TMB scores are typically used to predict whether a patient would respond to an immunotherapy treatment. Accordingly, the methods and systems provided herein can be used to distinguish variants of tumor or non-tumor origin as part of a TMB calculation, such as those described in PCT/US2019/042882. In another aspect, the present disclosure provides a method of classifying that a subject is a candidate for immunotherapy by determining whether the subject has a variant of tumor or non-tumor origin. In certain embodiments, the methods of the present discosure comprise administering one or more immunotherapies to the subject based on determining whether a variant is of tumor or non-tumor origin using the methods or systems disclosed herein alone or in combination with a method for determining a TMB score. In some embodiments, the immunotherapy comprises at least one checkpoint inhibitor antibody. In some embodiments, the immunotherapy comprises an antibody against PD-1, PD-2, PD-L1, PD-L2, CTLA-40, OX40, B7.1, B7He, LAG3, CD137, KIR, CCR5, CD27, or CD40. In some embodiments, the immunotherapy comprises administration of a pro-inflammatory cytokine against at least one tumor type. In some embodiments, the immunotherapy comprises administration of T cells against at least one tumor type. In some embodiments, the subject is administered a combination therapy (e.g. immunotherapy + PARPi + chemotherapies, etc.), among numerous other therapies further exemplified herein or otherwise known to those having ordinary skill in the art.

The methods and systems provided herein may be used to assess mutations for prognostic value concerning survival or response to treatment. For example, TP53 mutations may be assessed for prognostic and predictive value for treatment with an ALK inhibitor. The tumor/non-tumor origin determination of the variants analyzed herein may also be used for subject enrollment for select therpaies (e.g., TP53 drugs). Other applications of the methods and systems herein may be for analyzing mutations that are less well studied (e.g., FGFR2 mutations for FGFR inhibitors, or ERBB2 for ERBB2 inhibitors), where distinguishing between variants or tumor or non-tumor origin can provide confidence that the variants originate from a tumor or not. In certain embodiments, the methods and systems described herein may be used for monitoring molecular response by tracking tumor-only variants to determine the variant dynamic over time.

As additional therapies are developed for various diseases, interpretation of negative prediction will become increasingly complicated but critical in designing precision therapies.

**EXAMPLES**

**Example 1: Data Processing and Feature Engineering**

A model was developed to predict tumor or non-tumor origin for variants in an in-house database of over 180,000 plasma samples. The model was trained on multiple Guardant Health, Inc. and external public datasets with known tumor/non-tumor origin variants and tested on a cohort of samples with matched WBC and plasma cfDNA to validate the results. The model was applied to over 150,000 variants with data available both in-house and from external datasets, and resulted in a list of variants with associated probabilities of being non-tumor origin. At any point data can be added for re-training and re-classification of these variants.

Specifically, the development of this exemplary model implementation was achieved through multiple steps outlined below.

**1) Curating a truth dataset for training tumor and non-tumor variants**

To establish a training dataset for classifying variants as being of tumor or non-tumor origin, a truth list consisting of high confidence, well-established tumor variants was curated based on bona fide external sources of cancer variants, such as the National Comprehensive Cancer Network guidelines for cancer treatment, My Cancer Genome, MSKCC OncoKB (stratified by levels of evidence), literature, and other sources of known variants targetable by therapy or associated with therapeutic resistance. A truth list of bona fide variants associated with clonal hematopoiesis was curated from the literature and from samples sequenced from normal, healthy patients at Guardant Health, Inc.

**2) Sample Selection for aggregating variants**

The sample training set consisted of over > 180,000 in-house clinical and research samples, in-house healthy normals, and a combination of healthy normal and cancer data from external sources: Jaiswal et al. Age-related clonal hematopoiesis associated with adverse outcomes. N Engl J Med. 2014 Dec 25;371(26):2488-98. Zehir et al. Mutational landscape of metastatic cancer revealed from prospective clinical sequencing of 10,000 patients. Nat Med. 2017 Jun;23(6):703-713. Tate et al. COSMIC: the Catalogue Of Somatic Mutations In Cancer. Nucleic Acids Res. 2019 Jan 8;47(D1):D941-D947. Variants were identified by either gene and mutated amino acid or mutation cDNA, or by variant position and mutated nucleotides. To differentiate variants originating from a solid tissue tumor or from the blood, due to clonal hematopoiesis or hematological malignancy, all variants from healthy normals were pooled with samples from hematological malignancy-related cancer types.

**3) Feature engineering**

**A. Longitudinal mutant allele fraction**

Both the amplitude of the mutant allele fraction (MAF) and the dynamic behavior of a variant in a patient over time can be indicative of the variant origin as either tumor or biological noise. Variants for which there were at least two patients with samples from multiple time points (>1) were used for this analysis. For each variant and for each patient, the mean and variation in MAF (chi-square statistic, standard deviation) were calculated over multiple time points, which were then collapsed for each variant to calculate the overall mean of MAF means, representing the amplitude, and the mean or variation of variation, to represent the dynamic behavior over time. Together, the mean of means and the mean of variation represent a feature for subsequent analyses.

**B. Prevalence distributions of a variant across cancer types in plasma as an indicator of tumor/non-tumor origin**

The majority of true cancer variants have a tumor-specific profile that is driven by selection in the context of the tumor biology. In contrast, variants stemming from biological noise or clonal hematopoiesis should arise and thrive independently of the tissue cancer type. To determine the relative uniformity of the cancer type profile for a given variant, which may be indicative of a source of biological noise, the prevalence of each variant in plasma was calculated as a percentage of total samples for each cancer category as done previously for relative enrichment in plasma over tissue. This prevalence was used as an input to a logistic regression/random forest that would differentiate between background noise and non-random selection among cancer types.

**C. Proportion of samples in hematological malignancies as a predictor of tumor/non-tumor origin**

Variants that are frequently observed in hematological malignancies, such as leukemia and lymphoma, are specific to blood and not solid tumor variants. The proportion of total samples belonging to hematological malignancies was calculated for each variant and used as a single input feature to a logistic regression.

**D. Enrichment in plasma relative to tissue databases**

The strong enrichment of a variant in plasma databases compared to its relative prevalence for the same cancertype in tissue databases suggest that the variant might originate from clonal hematopoiesis and not from tumor. The relative ratio and p-value of the fold-change (Fisher's Exact Test) of prevalence in plasma compared to tissue was computed and normalized for each variant.

**Example 2: Variation in MAF across time points is associated with tumor and non-tumor origin. Non-tumor variants have a lower dispersion in MAF across time points.**

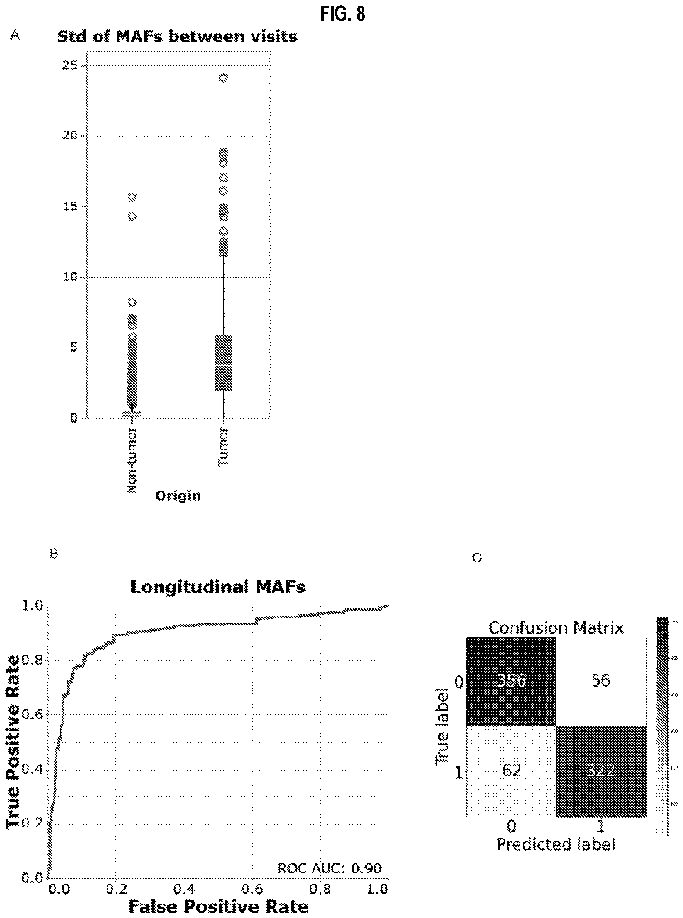

A dataset comprising over 180,000 plasma samples processed in-house was filtered for patients with at least 2 samples corresponding to different time points, and subsequently filtered for variants seen in at least 3 patients. For each patient, the mean of variant MAPs over time, as well as the standard deviation and chi-square p-value of the MAPs over time, were calculated for each variant, and summarized by the mean at the variant level across patients. The resulting mean of MAFs, standard deviations and chi-square p-values of MAFs were used as input features to a logistic regression model, representing both the amplitude and uniformity of the variant MAF across times points. FIG. 8, panel A, shows the separation in the mean standard deviation (SD) of percentages over time for the Non-tumor and Tumor class.

The dataset was filtered to a set of 2,509 variants with known tumor and non-tumor labels. Upsampling of a minority class was performed to ensure balanced labelling of classes. Resulting training and test set was 459 and 197 variants, respectively. Five-fold cross-validation was used to measure the performance of the model. Briefly, in K-fold cross-validation, the dataset is split into k smaller sets, wherein the following procedure is used for each of the k "folds":

a. A model is trained using k-1 of the folds as training data;

b. the resulting model is validated on the remaining part of the data (i.e., it is used as a test set to compute a performance measure such as accuracy).

The performance measure reported by k-fold cross-validation is then the average of the values computed in the loop.

ROC AUC was used to evaluate the performance of the classifier. In brief, ROC (Receiver Operating Characteristics) is a probability curve of true positive rate against the false positive rate at various threshold settings. AUC (or "Area under the ROC Curve") measures the two-dimensional area underneath the ROC curve, providing an aggregate measure of performance across all possible classification thresholds, thereby describing the probability that the model would classify a new variant correctly. AUC is commonly used for evaluating binary classifiers because it is invariant to scale (measures how well predictions are ranked, rather than their absolute values) and classification-thresholds, as it measures the quality of the model's predictions irrespective of what classification threshold is chosen. The ROC AUC of a single input feature in a logistic regression model was 90%, indicating high predictive value for this feature (FIG. 8, panel B). The accuracy for this model is: (TP+TN)/(TP+TN+FP+FN) = (322+356)/ (322+356+56+62) = 85.2%, where TP is true positive, TN is true negative, FP is false positive, and FN is false negative, and the value of 1 and 0 corresponds to tumor and non-tumor, respectively (FIG. 8, panel C).

**Example 3: Enrichment of a variant in plasma compared to tissue as an indicator of non-tumor origin**

The relative enrichment of variants in the plasma compared to tissue was performed as follows: samples with an annotated cancer type were aggregated into a superset of cancer categories and all samples without a cancer type were removed. The prevalence of an individual variant (number of samples with the variant in question for a cancer type divided by all samples in the cancer type) was calculated for each of the cancer categories in the plasma and tissue datasets, separately. The total number of plasma samples was over 180,000 while the total number of tissue samples was 291,847. The odds ratio of the prevalence in plasma compared to that in tissue and the corresponding p-value was calculated using the Fisher's Exact Test as the relative enrichment and used as a single input feature in subsequent analyses. FIG. 9, panels A and B, shows that known, well-established non-tumor variants have a higher ratio compared to tumor variants, regardless of the number of clinical samples observed (num_clin, panel A). The odds ratios and p-values were used as input features into a logistic regression model with Yeo-Johnson transformation applied to ensure a more Gaussian-like distribution, given by:

$$x_i^{(\lambda)} = \begin{cases} [(x_i + 1)^\lambda - 1]/\lambda & \text{if } \lambda \neq 0, x_i \geq 0, \\ \ln(x_i) + 1 & \text{if } \lambda = 0, x_i \geq 0 \\ -[(-x_i + 1)^{2-\lambda} - 1]/(2 - \lambda) & \text{if } \lambda \neq 2, x_i < 0, \\ -\ln(-x_i + 1) & \text{if } \lambda = 2, x_i < 0 \end{cases}$$

The performance of this model is shown to be a ROC AUC of 81% (FIG. 9, panel C). The accuracy for this model is: (TP+TN)/(TP+TN+FP+FN) = (2333+3135)/( 2333+3135+561+1295) = 5,468/7,324 (74.6%), where TP is true positive, TN is true negative, FP is false positive, and FN is false negative (FIG. 9, panel C).

**Example 4: Non-tumor specific variants show greater uniformity in prevalence across cancer types compared to known tumor drivers**

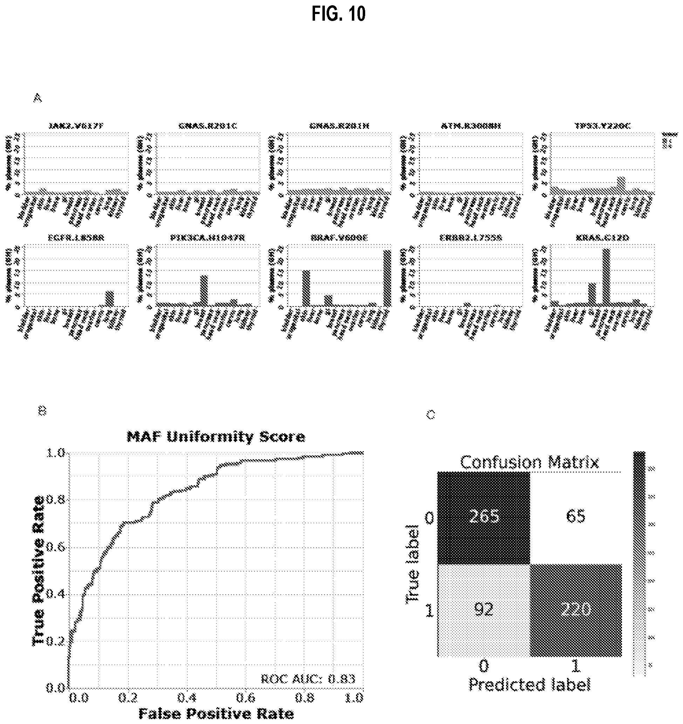

Prevalence of each variant was calculated per cancer type as described in Example 2 (number of samples with the variant divided by total number of samples in that cancer type). For variants of non-tumor origin, prevalence in cancer types is uniformly low (FIG. 10, panel A, upper panel), compared to tumor-specific variants, where variants can show specific prevalence profiles driven by selection and biology of the tumor (FIG. 10, panel A, bottom panel). The prevalence of each variant for each individual cancer type was used as an input feature to a logistic regression model. The ROC AUC in the test set was 83% (FIG. 10, panel B). The accuracy of the model was 75.5%, based on the accuracy formula, (TP+TN)/(TP+TN+FP+FN) = (220+265)/( 220+265+65+92), where TP is true positive, TN is true negative, FP is false positive, and FN is false negative (FIG. 10, panel C).

**Example 5: High proportion of leukemia/lymphoma samples supporting a variant is highly indicative of non-tumor status**

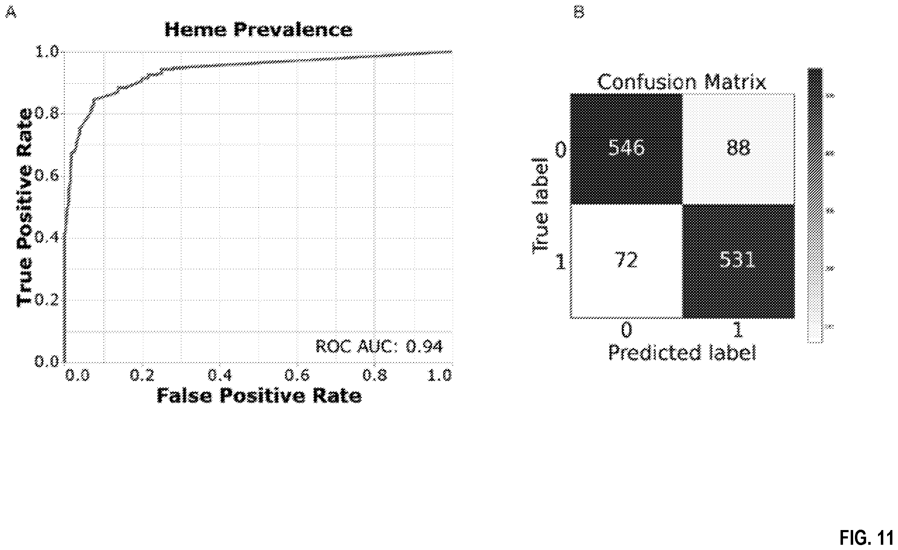

Using the tissue dataset from COSMIC comprising 281,718 samples, the total number of samples for a given variant in leukemia/lymphoma/hematological malignancies or healthy individuals compared to all samples containing that variant across all cancer types were calculated. The proportion of samples with the variant in leukemia/lymphoma indicates the likelihood of that variant appearing in the blood; the higher the probability to originate from blood, the lower probability it is a tumor variant. Thus, this proportion in "heme" (hematological malignancy) was used as a single input feature into a logistic regression model. The ROC AUC of the performance of the model was 0.94% (FIG. 11, panel A), indicating high predictive value for this feature. The accuracy for this model is: (TP+TN)/(TP+TN+FP+FN) = (531+546)/( 531+546+88+72) = 87.1% (FIG. 11, panel B), where TP is true positive, TN is true negative, FP is false positive, and FN is false negative.

**Example 6: Random forest machine learning algorithm**

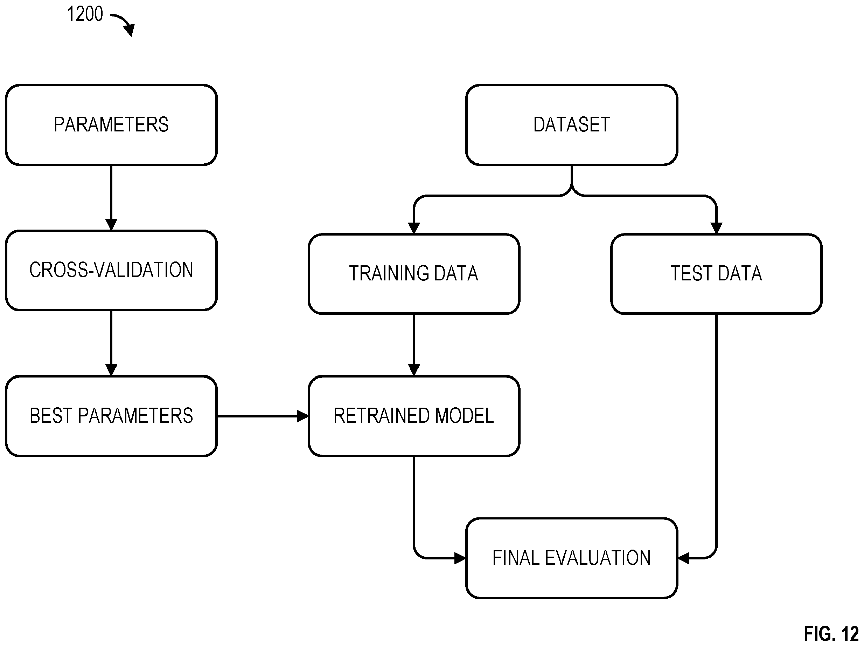

Using the features described above, a random forest model was trained to classify somatic mutations detected in plasma according to their source of origin. Briefly, random forest is an ensemble learning method widely used for classification, regression and other methods of supervised learning. A random forest used for classification is a meta estimator that fits a number of decision tree classifiers at training time on various sub-samples of the dataset, and uses averaging to determine the predicted class in order to improve the predictive accuracy and control for over-fitting of the model. Here, a random forest model was trained to classify mutations detected in plasma as derived from a tumor or non-tumor origin, where the sub-sample size is always the same as the original input sample size but the samples were drawn with replacement. Implementation of random forest classifier from Scikit-learn machine learning library was used (retrieved from the Internet <URL: https://scikit-learn.org> [retrieved on 2019-07-25). Training of the model (model 1200) is described according to the Machine Learning Modeling Flowchart (see, FIG. 12).

Stratified sampling was used for splitting the original dataset into train (80% of the entire dataset) and test (20% of the entire dataset) sets. This type of sampling and splitting was chosen to ensure that the train and test sets have approximately the same percentage of samples of each target class as the complete set.

The best set of model hyper-parameters was determined by grid search techniques. Hyper-parameters are parameters that are not directly learned within estimators (for Random Forests these parameters are the maximum depth of the decision tree, the number of trees in the forest, etc.). The hyper-parameter space was studied for the best cross validation score by grid search, which exhaustively checks all the possible combinations of parameter values, evaluates the model performance, and retains the best combination of parameters. A 10-fold cross-validation was used to measure performance of the model giving a set of hyper-parameters. In K-fold cross-validation, the dataset was split into k smaller sets. The following procedure was used for each of the k "folds":

> a. A model is trained using k-1 of the folds as training data;
> b. the resulting model is validated on the remaining part of the data (i.e., it is used as a test set to compute a performance measure such as accuracy).

The performance measure reported by k-fold cross-validation is then the average of the values computed in the loop.

The optimal set of parameters was determined to be:

- The maximum depth of the tree: 2.
- The number of estimators in the forest: 300.

The model was finally retrained using the optimal set of parameters identified during the previous steps. The final performance of the model was evaluated using the test set.

The trained model was used to determine a tumor or non-tumor prediction across all variants previously observed in-house to obtain a list of high confidence tumor or non-tumor variants.

**Example 7: Random forest classifier (Ensemble Model) with 4 input features to predict tumor/non-tumor status**

A set of 2,509 variants with known tumor and non-tumor labels were used to train and evaluate the performance of a random forest classifier. The input labels were divided into a training and test consisting of 1997 and 500 variants, respectively. To build the model, observed data across over 110,000 samples were pre-processed into 4 features as described in Examples 2, 3, 4 and 5 as well as the genes for the variants input with one-hot encoding. Grid Search, a process that scans and iterates through every hyperparameter combination to find the optimal configurations for the model, was performed on the pre-processed dataset using a manually specified subset of hyperparameters that were estimated to be appropriate for this model: 2, 3 and 4 for the depth of trees, and 50, 100, 200, 300 and 500 as the number of estimators. The optimal hyperparameters for this dataset, as determined by Grid Search, was determined to be max depth of 2 and 300 estimators, as well as a max depth of 3 and 500 estimators.

To evaluate the impact of each set of hyperparameters on the random forest model, 5-fold cross validation was performed across the input dataset with known tumor and non-tumor labels.

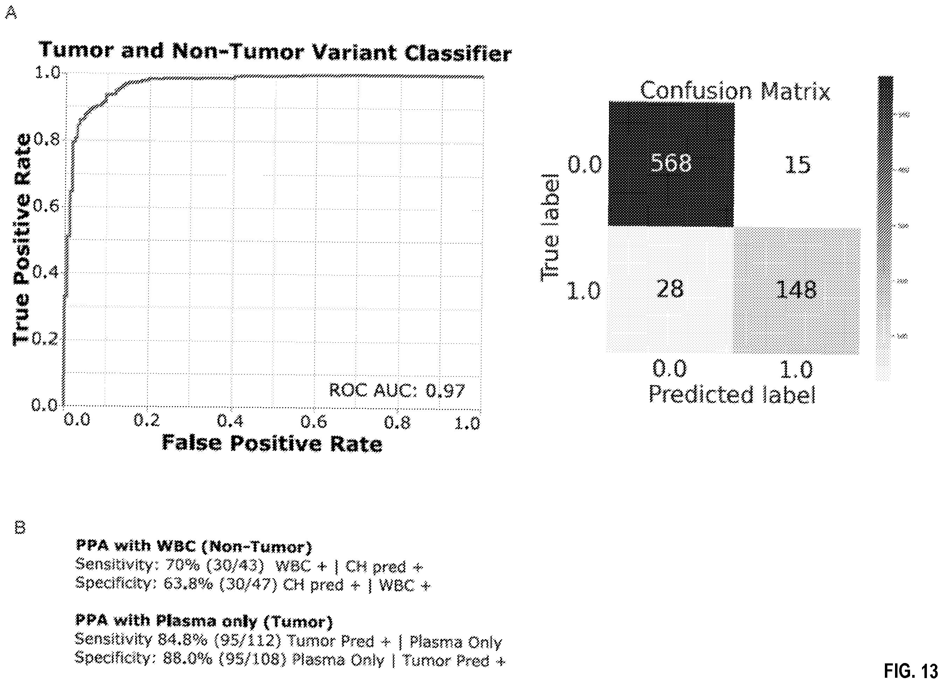

FIG. 13, panel A shows that the performance of the classifier trained with a max depth of 2 and 300 estimators. The ROC AUC was 97% on the training dataset, demonstrating a high probability of classifying a new variant correctly. To estimate the accuracy of the classifier, a confusion matrix was created for predicted and known tumor labels on the test dataset. The accuracy of the model is based on the accuracy formula, $(TP+TN)/(TP+TN+FP+FN) = (148+568)/(148+568+15+28) = 94.0\%$, where TP is true positive, TN is true negative, FP is false positive, and FN is false negative. Therefore, the model was able to classify 94.0% of variants correctly on this test dataset. FIG. 13, panel B, is the performance of the classifier on a validation dataset with vairants confirmed only in plasma (tumor) or in the white blood cell (WBC) fraction (non-tumor).

The performance of the classifier trained with a max depth of 3 and 500 estimators. The ROC AUC was also 91% on the training dataset, with an accuracy $((TP+TN)/(TP+TN+FP+FN))$ of $(115+309)/(115+309+45+31) = 354/500 = 70.8\%$, where TP is true positive, TN is true negative, FP is false positive, and FN is false negative. Given the lower accuracy and equivalent ROC AUC despite a greater depth and number of trees/estimators, it was concluded that a lower depth and fewer estimators (2, 300, respectively) would be better hyperparameters for this model.

**Example 8: Logistic regression classifier (Ensemble model) with 4 input features to predict tumor/non-tumor status**

A training and test dataset with known tumor/non-tumor labels was created and an input dataset with features were pre-processed as described in EXAMPLE 7. For the logistic regression model, multiple feature scaling methods were applied to normalize the data, ensure Gaussian-like distribution, stabilize variance and minimize skewness. Given that the input features have different units, zero-mean and unit-variance were also used to further normalize to ensure that the features were on the same scale and centered at 0 with a standard deviation of 1, thus preventing any bias due to different scales or range of values. Polynomial features were applied to generate a new feature matrix consisting of all polynomial combinations of the features. An input of 2 degrees was used to prevent overfitting.

Five-fold cross validation was performed across the input dataset with known tumor and non-tumor labels. The ROC AUC was 92% on the test data. The accuracy of our model $(TP+TN)/(TP+TN+FP+FN) =$

(103+334)/(103+334+20+43) = 437/500 (87.4%), where TP is true positive, TN is true negative, FP is false positive, and FN is false negative, indicating this model is able to classify 87.4% of variants correctly on this test dataset.

**EXAMPLE 9: Performance of tumor/non-tumor classifications on a paired white blood cell and plasma cohort**

A subsequent validation was performed on a set of 38 paired plasma and white blood cell (WBC) fractions, which had been previously sequenced and analyzed in house and discussed further in Yen et al. Analysis of clonal hematopoiesis in cell-free DNA of advanced cancer patients (Poster #5396, AACR 2019), and were not used in the training of the model. Briefly, variants that were detected exclusively in the plasma fraction were labelled as tumor while variants detected in both plasma and in the WBC fraction were labelled as non-tumor. A subset of variants detected in the plasma-WBC set (98/648, 15%) were examined to predict a tumor/non-tumor status, the accuracy of which could be determined based on concordance with the plasma-WBC dataset. Compared to the ensemble random forest classifier 5-fold cross validation performance (ROC AUC of 97%), 95/108 (88.0%) of variants predicted as tumor origin were confirmed as tumor origin based on the plasma-WBC data. As the model was optimized for sensitivity for accuracy of tumor variants, sensitivity of calling tumor was slightly lower, where 95/112 (84.8%) of confirmed tumor variants were predicted as tumor, and specificity for non-tumor predictions was also lower (30/43, 70.0%), likely owing to the smaller limited training data-set for non-tumor variants.

**Table 1. Concordance of tumor and non-tumor predictions with paired plasma and WBC samples**

|  |  | Predicted | |  |
| --- | --- | --- | --- | --- |
|  |  | Tumor | Non-tumor | Total |
| Confirmed | Tumor | 30 | 13 | 43 |
|  | Non-tumor | 17 | 95 | 112 |
|  | Total | 47 | 108 | 155 |

**Conclusions**

A data-driven and bioinformatics approach to classifying tumor and non-tumor variants in cfDNA is important for the accurate reporting of tumor variants that inform on therapy selection in clinical diagnostic settings and for the appropriate identification of biomarkers with predictive or prognostic alue in clinical studies. The number of new tumor and non-tumor vairants that can be classified using this model should improve along with the perofrmace of the classifier as sequenced cfDNA samples are accumulated over time. A highly sensitive and specific tumor and non-tumor variant classifier reduces the reliance of accurate ctDNA detection on additional paired tissue or white blood cell fractions, which are difficult to obtain from patients and complicates the sample processing workflow.

If different versions of a sequence are associated with an accession number at different times, the version associated with the accession number at the effective filing date of this application is meant. The effective filing date means the earlier of the actual filing date or filing date of a priority application referring to the accession number if applicable. Likewise if different versions of a publication, website or the like are published at different times, the version most recently published at the effective filing date of the application is meant unless otherwise indicated. Any feature, step, element, embodiment, or aspect of the disclosure can be used in combination with any other unless specifically indicated otherwise. Although the present disclosure has been described in some detail by way of illustration and example for purposes of clarity and understanding, it will be apparent that certain changes and modifications may be practiced within the scope of the appended claims.

**Claims**

1. A method of differentiating tumor and non-tumor origin nucleic acid variants in a cell-free nucleic acid (cfNA) sample obtained from a test subject at least partially using a computer, the method comprising:

   generating or providing, by the computer, at least one tumor variant dataset comprising a population of reference tumor-related genetic variants, wherein the tumor variant dataset comprises frequency of observance data among reference samples that comprises reference bodily fluid samples and reference non-bodily fluid samples for one or more tumor-related genetic variants in the population of reference tumor-related genetic variants, and wherein the reference samples are obtained from a single reference subject and/or from different reference subjects having an identical cancer type;

determining, by the computer, one or more ratios of the frequency of observance data between the reference samples for one or more tumor-related genetic variants in the population of reference tumor-related genetic variants to produce at least one mutant allele fraction (MAF) variance and/or relative prevalence dataset, wherein uniformity of prevalence across cancer types, longitudinal MAF variation over time, and/or proportion in hematological cancers is utilized in conjunction with the ratios of the frequency of observance data;

generating, by the computer, at least one set of probabilities of non-tumor origin from the MAF variance and/or relative prevalence dataset; and,

using the set of probabilities of non-tumor origin to differentiate nucleic acid variants detected in the cfNA sample obtained from the test subject as being tumor origin nucleic acid variants or non-tumor origin nucleic acid variants.

2. The method of claim 1, wherein the relative prevalence dataset is produced based on

(a) determining, by the computer, relative prevalence of one or more tumor-related genetic variants observed in one or more reference bodily fluid samples compared to one or more reference non-bodily fluid samples; or
(b) determining, by the computer, a variation in a mutant allele fraction (MAF) value, and/or at least one statistic related thereto, for at least two different time points for each of one or more tumor-related and/or non-tumor-related genetic variants.

3. The method of claim 1 or claim 2, wherein the at least one set of probabilities of non-tumor origin is generated from the relative prevalence dataset, and wherein differentiating the tumor and non-tumor origin nucleic acid variants in the cfNA sample obtained from the test subject comprises:

classifying, by the computer, at least a first nucleic acid variant detected in the cfNA sample obtained from the test subject as being a tumor origin nucleic acid variant when a prevalence of the first nucleic acid variant detected in the cfNA sample is less than a threshold of probability from the set of probabilities of non-tumor origin and
classifying, by the computer, at least a second nucleic acid variant detected in the cfNA sample obtained from the test subject as being a non-tumor origin nucleic acid variant when a prevalence of the second nucleic acid variant detected in the cfNA sample is greater than a threshold of probability from the set of probabilities of non-tumor origin.

4. The method of any one preceding claim,

(a) comprising identifying genetic variants present in the cfNA sample from sequencing reads originating from cfNA molecules in the cfNA sample, for example wherein the sequencing reads are obtained from targeted segments of the cfNA molecules in the cfNA sample;
(b) wherein the population of reference tumor-related genetic variants are obtained from the reference samples; and/or
(c) wherein the tumor variant dataset comprises frequency of observance data among reference samples of a given cancer type for one or more tumor-related genetic variants in the population of reference tumor-related genetic variants.

5. The method of any one preceding claim, comprising training a machine learning model using at least a portion of the population of tumor-related genetic variants to produce a trained machine learning model, wherein the tumor-origin nucleic acid variants and non-tumor origin nucleic acid variants detected in the cfNA sample obtained from the test subject are differentiated from one another using the trained machine learning model, optionally:

(a) wherein the machine learning model is trained using one or more of: logistic regression, probit regression, decision trees, random forests, gradient boosting, support vector machines, K-nearest neighbors, and a neural network;
(b) comprising randomly splitting the tumor variant dataset into a training dataset and a test dataset; and/or
(c) wherein the training dataset comprises about 80% of the tumor variant dataset and the test dataset comprises about 20% of the tumor variant dataset.

6. The method of any one preceding claim,

(a) comprising using a threshold of probability of at least about a 30th percentile for a given genetic variant as a cut-off for classification;

(b) comprising performing logistic regression on at least one of the ratios to obtain a given probability of non-tumor origin;

(c) wherein the tumor variant dataset comprises mutant allele fraction data observed among reference samples for one or more tumor-related genetic variants in the population of reference tumor-related genetic variants; and/or

(d) comprising normalizing the tumor variant dataset using one or more data normalization techniques, for example wherein the data normalization techniques comprise min-max normalization and/or z-score normalization.

7. The method of any one preceding claim, wherein the reference non-bodily fluid samples comprise reference tumor tissue samples and/or reference white blood cell samples; optionally wherein:

(a) a ratio of frequency of observance data of a given genetic variant in the reference bodily fluid samples relative to frequency of observance data of the given genetic variant in the reference non-bodily fluid samples that is greater than one (1.0) indicates that the given genetic variant is likely a non-tumor origin nucleic acid variant, wherein the reference non-bodily fluid samples comprise reference tumor tissue samples; or

(b) a ratio of frequency of observance data of a given genetic variant in the reference bodily fluid samples relative to frequency of observance data of the given genetic variant in the reference non-bodily fluid samples that is less than one (1.0) indicates that the given genetic variant is likely a non-tumor origin nucleic acid variant, wherein the reference non-bodily fluid samples comprise reference white blood cell samples.

8. The method of any one preceding claim, wherein the set of probabilities of non-tumor origin comprise at least one set of probabilities of clonal hematopoiesis origin.

9. The method of any one preceding claim, comprising selecting one or more therapies to treat a cancer type when one or more tumor origin nucleic acid variants associated with the cancer type are detected in the cfNA sample obtained from the test subject.

10. The method of any one preceding claim, wherein the cancer type is selected from the group consisting of: bilary tract cancer, bladder cancer, transitional cell carcinoma, urothelial carcinoma, brain cancer, gliomas, astrocytomas, breast carcinoma, metaplastic carcinoma, cervical cancer, cervical squamous cell carcinoma, rectal cancer, colorectal carcinoma, colon cancer, hereditary nonpolyposis colorectal cancer, colorectal adenocarcinomas, gastrointestinal stromal tumors (GISTs), endometrial carcinoma, endometrial stromal sarcomas, esophageal cancer, esophageal squamous cell carcinoma, esophageal adenocarcinoma, ocular melanoma, uveal melanoma, gallbladder carcinomas, gallbladder adenocarcinoma, renal cell carcinoma, clear cell renal cell carcinoma, transitional cell carcinoma, urothelial carcinomas, wilms tumor, leukemia, acute lymphocytic leukemia (ALL), acute myeloid leukemia (AML), chronic lymphocytic (CLL), chronic myeloid (CML), chronic myelomonocytic (CMML), liver cancer, liver carcinoma, hepatoma, hepatocellular carcinoma, cholangiocarcinoma, hepatoblastoma, Lung cancer, non-small cell lung cancer (NSCLC), mesothelioma, B-cell lymphomas, non-Hodgkin lymphoma, diffuse large B-cell lymphoma, Mantle cell lymphoma, T cell lymphomas, non-Hodgkin lymphoma, precursor T-lymphoblastic lymphoma/leukemia, peripheral T cell lymphomas, multiple myeloma, nasopharyngeal carcinoma (NPC), neuroblastoma, oropharyngeal cancer, oral cavity squamous cell carcinomas, osteosarcoma, ovarian carcinoma, pancreatic cancer, pancreatic ductal adenocarcinoma, pseudopapillary neoplasms, acinar cell carcinomas, prostate cancer, prostate adenocarcinoma, skin cancer, melanoma, malignant melanoma, cutaneous melanoma, small intestine carcinomas, stomach cancer, gastric carcinoma, gastrointestinal stromal tumor (GIST), uterine cancer, and uterine sarcoma.

11. The method of any one preceding claim, wherein

(a) the reference tumor-related genetic variants are selected from the group consisting of: single nucleotide variants (SNVs), insertions or deletions (indels), copy number variants (CNVs), fusions, transversions, translocations, frame shifts, duplications, repeat expansions, and epigenetic variants; and/or

(b) the reference samples comprise at least about 25, at least about 50, at least about 100, at least about 200, at least about 300, at least about 400, at least about 500, at least about 600, at least about 700, at least about 800, at least about 900, at least about 1,000, at least about 5,000, at least about 10,000, at least about 15,000, at least about 20,000, at least about 25,000, at least about 30,000, or more bodily fluid and/or non-bodily fluid samples.

**12.** The method of any one preceding claim, wherein

(a) the cfNA sample comprises cell-free deoxyribonucleic acid (cfDNA), and/or wherein the cfNA sample comprises cell-free ribonucleic acid (cfRNA);

(b) the test subject is a mammalian subject, for example wherein the test subject is a human subject;

(c) the reference bodily fluid samples comprise plasma samples, and/or wherein the reference bodily fluid samples comprise serum samples; and/or

(d) the reference non-bodily fluid samples comprise cell samples, and/or wherein the reference non-bodily fluid samples comprise tissue samples.

**13.** A system, comprising a controller comprising, or capable of accessing, computer readable media comprising non-transitory computer-executable instructions which, when executed by at least one electronic processor perform at least: the method of any one of the preceding claims.

**14.** The system of claim 13, comprising

(a) a nucleic acid sequencer operably connected to the controller, which nucleic acid sequencer is configured to provide sequencing reads originating from cfNA molecules in the cfNA samples; and/or wherein the nucleic acid sequencer or another system component is configured to group sequence reads generated by the nucleic acid sequencer into families of sequence reads, each family comprising sequence reads generated from a given cfNA molecule in the cfNA samples;

(b) a database operably connected to the controller, which database comprises one or more therapies indexed to the tumor origin nucleic acid variants;

(c) a sample preparation component operably connected to the controller, which sample preparation component is configured to prepare the cfNA molecules in the cfNA samples to be sequenced by the nucleic acid sequencer;

(d) a nucleic acid amplification component operably connected to the controller, which nucleic acid amplification component is configured to amplify at least targeted segments of the cfNA molecules in the cfNA samples; and/or

(e) a material transfer component operably connected to the controller, which material transfer component is configured to transfer one or more materials between at least the nucleic acid sequencer and the sample preparation component.

**15.** A computer readable media comprising non-transitory computer-executable instructions which, when executed by at least one electronic processor perform at least: the method of any one of claims 1-12.

**Patentansprüche**

**1.** Verfahren zur Unterscheidung von Nukleinsäurevarianten von Tumor- bzw. Nichttumorursprung in einer zellfreien Nukleinsäure (cfNA)-Probe, die aus einem Testsubjekt gewonnen wurde, zumindest teilweise unter Verwendung eines Computers, wobei das Verfahren umfasst:

Erzeugen oder Bereitstellen von mindestens einem Tumorvariantendatensatz, der eine Population von tumorbezogenen genetischen Referenzvarianten umfasst, durch den Computer, wobei der Tumorvariantendatensatz Beobachtungshäufigkeitsdaten unter Referenzproben, die Referenz-Körperflüssigkeitsproben und Referenz-Nichtkörperflüssigkeitsproben umfassen, für eine oder mehrere tumorbezogene genetische Varianten in der Population von tumorbezogenen genetischen Referenzvarianten umfasst, und wobei die Referenzproben aus einem einzelnen Referenzsubjekt und/oder aus verschiedenen Referenzsubjekten mit identischem Krebstyp gewonnen werden;

Bestimmen eines oder mehrerer Verhältnisse der Beobachtungshäufigkeitsdaten zwischen den Referenzproben für eine oder mehrere tumorbezogene genetische Varianten in der Population von tumorbezogenen genetischen Referenzvarianten durch den Computer zum Erstellen mindestens eines Varianz- und/oder Relative-Prävalenz-Datensatzes für die Mutanten-Allel-Fraktion (MAF), wobei die Einheitlichkeit der Prävalenz über Krebstypen hinweg, die longitudinale MAF-Variation im Zeitverlauf und/oder der Anteil hämatologischer Krebsarten in Verbindung mit den Verhältnissen der Beobachtungshäufigkeitsdaten verwendet wird;

Generieren von mindestens einem Satz von Wahrscheinlichkeiten des Nichttumorursprungs aus dem MAF-Varianz- und/oder Relative-Prävalenz-Datensatz, durch den Computer; und

Verwenden des Satzes von Wahrscheinlichkeiten des Nichttumorursprungs zur Unterscheidung von Nukleinsäurevarianten, die in der aus dem Testsubjekt gewonnenen cfNA-Probe nachgewiesen wurden, als Tumorursprung-Nukleinsäurevarianten oder Nichttumorursprung-Nukleinsäurevarianten.

2.  Verfahren nach Anspruch 1, wobei der Relative-Prävalenz-Datensatz erstellt wird auf der Grundlage von

    (a) der computergestützten Bestimmung der relativen Prävalenz einer oder mehrerer tumorbezogener genetischer Varianten, die in einer oder mehreren Referenz-Körperflüssigkeitsproben beobachtet wurden, im Vergleich zu einer oder mehreren Referenz-Nichtkörperflüssigkeitsproben; oder
    (b) der computergestützten Bestimmung einer Variation eines Mutanten-Allel-Fraktion (MAF)-Werts und/oder mindestens einer damit verbundenen Statistik für mindestens zwei verschiedene Zeitpunkte für jede von einer oder mehreren tumorbezogenen und/oder nichttumorbezogenen genetischen Variante(n).

3.  Verfahren nach Anspruch 1 oder Anspruch 2, wobei der mindestens eine Satz von Wahrscheinlichkeiten des Nichttumorursprungs aus dem Relative-Prävalenz-Datensatz generiert wird und wobei das Unterscheiden der Tumor- und Nichttumorursprung-Nukleinsäurevarianten in der aus dem Testsubjekt gewonnenen cfNA-Probe Folgendes umfasst:

    die computergestützte Klassifizierung von mindestens einer ersten Nukleinsäurevariante, die in der aus dem Testsubjekt gewonnenen cfNA-Probe nachgewiesen wurde, als eine Tumorursprung-Nukleinsäurevariante, wenn eine Prävalenz der ersten Nukleinsäurevariante, die in der cfNA-Probe nachgewiesen wurde, kleiner ist als ein Wahrscheinlichkeitsschwellenwert aus dem Satz von Wahrscheinlichkeiten des Nichttumorursprungs, und
    die computergestützte Klassifizierung von mindestens einer zweiten Nukleinsäurevariante, die in der aus dem Testsubjekt gewonnenen cfNA-Probe nachgewiesen wurde, als eine Nichttumorursprung-Nukleinsäurevariante, wenn eine Prävalenz der zweiten Nukleinsäurevariante, die in der cfNA-Probe nachgewiesen wurde, größer ist als ein Wahrscheinlichkeitsschwellenwert aus dem Satz von Wahrscheinlichkeiten des Nichttumorursprungs.

4.  Verfahren nach einem der vorhergehenden Ansprüche,

    (a) umfassend das Identifizieren von in der cfNA-Probe vorhandenen genetischen Varianten aus Sequenzierungsablesungen, die von cfNA-Molekülen in der cfNA-Probe stammen, beispielsweise wobei die Sequenzierungsablesungen aus angezielten Segmenten der cfNA-Moleküle in der cfNA-Probe gewonnen werden;
    (b) wobei die Population von tumorbezogenen genetischen Referenzvarianten aus den Referenzproben gewonnen wird; und/oder
    (c) wobei der Tumorvarianten-Datensatz Beobachtungshäufigkeitsdaten unter Referenzproben eines jeweiligen Krebstyps für eine oder mehrere tumorbezogene genetische Varianten in der Population von tumorbezogenen genetischen Referenzvarianten umfasst.

5.  Verfahren nach einem der vorhergehenden Ansprüche, umfassend das Trainieren eines Machine-Learning-Modells unter Verwendung von mindestens einem Teil der Population von tumorbezogenen genetischen Varianten, um ein trainiertes Machine-Learning-Modell zu erstellen, wobei die in der aus dem Testsubjekt gewonnenen cfNA-Probe nachgewiesenen Tumorursprung-Nukleinsäurevarianten und Nichttumorursprung-Nukleinsäurevarianten unter Verwendung des trainierten Machine-Learning-Modells voneinander unterschieden werden, optional:

    (a) wobei das Machine-Learning-Modell mithilfe eines oder mehrerer der Folgenden trainiert wird: logistische Regression, Probit-Regression, Entscheidungsbäume, Random-Forests, Gradientenverstärkung, Support-Vector-Maschinen, K-Nearest-Neighbors und einem neuronalen Netzwerk;
    (b) umfassend das zufällige Aufteilen des Tumorvarianten-Datensatzes in einen Trainingsdatensatz und einen Testdatensatz; und/oder
    (c) wobei der Trainingsdatensatz etwa 80 % des Tumorvarianten-Datensatzes und der Testdatensatz etwa 20 % des Tumorvarianten-Datensatzes umfasst.

6.  Verfahren nach einem der vorhergehenden Ansprüche,

    (a) umfassend die Verwendung eines Wahrscheinlichkeitsschwellenwerts von mindestens etwa einem 30. Perzentil für eine jeweilige genetische Variante als Cut-off für die Klassifizierung;
    (b) umfassend die Durchführung einer logistischen Regression auf mindestens einem der Verhältnisse, um eine jeweilige Wahrscheinlichkeit des Nichttumorursprungs zu erhalten;
    (c) wobei der Tumorvarianten-Datensatz Mutanten-Allel-Fraktion-Daten umfasst, die unter Referenzproben für eine oder mehrere tumorbezogene genetische Varianten in der Population von tumorbezogenen genetischen Referenzvarianten beobachtet wurden; und/oder

(d) umfassend die Normalisierung des Tumorvarianten-Datensatzes mithilfe einer oder mehrerer Datennormalisierungstechniken, beispielsweise wobei die Datennormalisierungstechniken eine Min-Max-Normalisierung und/oder eine Z-Score-Normalisierung umfassen.

7. Verfahren nach einem der vorhergehenden Ansprüche, wobei die Referenz-Nichtkörperflüssigkeitsproben Referenz-Tumorgewebeproben und/oder Referenzproben weißer Blutkörperchen umfassen; optional wobei:

   (a) ein Verhältnis der Beobachtungshäufigkeitsdaten einer jeweiligen genetischen Variante in den Referenz-Körperflüssigkeitsproben relativ zu Beobachtungshäufigkeitsdaten der jeweiligen genetischen Variante in den Referenz-Nichtkörperflüssigkeitsproben, das größer als eins (1,0) ist, anzeigt, dass die jeweilige genetische Variante wahrscheinlich eine Nichttumorursprung-Nukleinsäurevariante ist, wobei die Referenz-Nichtkörperflüssigkeitsproben Referenz-Tumorgewebeproben umfassen; oder

   (b) ein Verhältnis der Beobachtungshäufigkeitsdaten einer jeweiligen genetischen Variante in den Referenz-Körperflüssigkeitsproben relativ zu Beobachtungshäufigkeitsdaten der jeweiligen genetischen Variante in den Referenz-Nichtkörperflüssigkeitsproben, das kleiner als eins (1,0) ist, anzeigt, dass die jeweilige genetische Variante wahrscheinlich eine Nichttumorursprung-Nukleinsäurevariante ist, wobei die Referenz-Nichtkörperflüssigkeitsproben Referenzproben weißer Blutkörperchen umfassen.

8. Verfahren nach einem der vorhergehenden Ansprüche, wobei der Satz von Wahrscheinlichkeiten des Nichttumorursprungs mindestens einen Satz von Wahrscheinlichkeiten des klonalen hämatopoetischen Ursprungs umfasst.

9. Verfahren nach einem der vorhergehenden Ansprüche, umfassend die Auswahl einer oder mehrerer Therapien zur Behandlung eines Krebstyps, wenn in der aus dem Testsubjekt gewonnenen cfNA-Probe eine oder mehrere mit dem Krebstyb assoziierte Tumorursprung-Nukleinsäurevarianten nachgewiesen werden.

10. Verfahren nach einem der vorhergehenden Ansprüche, wobei der Krebstyp ausgewählt ist aus der Gruppe bestehend aus: Gallenwegskrebs, Blasenkrebs, Übergangszellen-Karzinom, Urothelkarzinom, Hirntumor, Gliomen, Astrozytomen, Brustkarzinom, metaplastischem Karzinom, Gebärmutterhalskrebs, zervikalem Plattenepithelkarzinom, Rektumkarzinom, kolorektalem Karzinom, Kolonkrebs, hereditärem nichtpolypösen kolorektalen Krebs, kolorektalen Adenokarzinomen, gastrointestinalen Stromatumoren (GIST), Endometriumkarzinom, endometrialen Stromasarkomen, Speiseröhrenkrebs, Speiseröhrenplattenepithelkarzinom, Speiseröhrenadenokarzinom, okulärem Melanom, Aderhautmelanom, Gallenblasenkarzinomen, Gallenblasenadenokarzinom, Nierenzellkarzinom, klarzelligem Nierenzellkarzinom, Übergangszellkarzinom, Urothelkarzinomen, Wilms-Tumor, Leukämie, akuter lymphatischer Leukämie (ALL), akuter myeloischer Leukämie (AML), chronischer lymphatischer (CLL), chronischer myeloischer (CML), chronischer myelomonozytischer Leukämie (CMML), Leberkrebs, Leberkarzinom, Hepatom, hepatozellulärem Karzinom, Cholangiokarzinom, Hepatoblastom, Lungenkrebs, nicht-kleinzelligem Lungenkrebs (NSCLC), Mesotheliom, B-Zell-Lymphomen, Non-Hodgkin-Lymphom, diffusem großzelligen B-Zell-Lymphom, Mantelzell-Lymphom, T-Zell-Lymphomen, Non-Hodgkin-Lymphom, Vorläufer-T-lymphoblastischem Lymphom/Leukämie, peripheren T-Zell-Lymphomen, Multiplem Myelom, Nasopharynxkarzinom (NPC), Neuroblastom, Oropharynxkrebs, Plattenepithelkarzinomen der Mundhöhle, Osteosarkom, Eierstockkarzinom, Bauchspeicheldrüsenkrebs, Pankreasgangadenokarzinom, pseudopapillären Neoplasien, Azinuszellkarzinomen, Prostatakrebs, Prostataadenokarzinom, Hautkrebs, Melanom, malignem Melanom, kutanem Melanom, Dünndarmkarzinomen, Magenkrebs, Magenkarzinom, gastrointestinalem Stromatumor (GIST), Gebärmutterkrebs und Gebärmuttersarkom.

11. Verfahren nach einem der vorhergehenden Ansprüche, wobei

   (a) die tumorbezogenen genetischen Referenzvarianten aus der Gruppe bestehend aus Einzelnukleotidvarianten (SNVs), Insertionen oder Deletionen (Indels), Kopienzahlvarianten (CNVs), Fusionen, Transversionen, Translokationen, Leserasterverschiebungen, Duplikationen, Wiederholungseinheit-Expansionen und epigenetischen Varianten ausgewählt werden; und/oder

   (b) die Referenzproben mindestens etwa 25, mindestens etwa 50, mindestens etwa 100, mindestens etwa 200, mindestens etwa 300, mindestens etwa 400, mindestens etwa 500, mindestens etwa 600, mindestens etwa 700, mindestens etwa 800, mindestens etwa 900, mindestens etwa 1.000, mindestens etwa 5.000, mindestens etwa 10.000, mindestens etwa 15.000, mindestens etwa 20.000, mindestens etwa 25.000, mindestens etwa 30.000 oder mehr Körperflüssigkeits- und/oder Nichtkörperflüssigkeitsproben umfassen.

**12.** Verfahren nach einem der vorhergehenden Ansprüche, wobei

(a) die cfNA-Probe zellfreie Desoxyribonukleinsäure (cfDNA) umfasst und/oder wobei die cfNA-Probe zellfreie Ribonukleinsäure (cfRNA) umfasst;
(b) das Testsubjekt ein Säugetier ist, wobei das Testsubjekt beispielsweise ein Mensch ist;
(c) die Referenz-Körperflüssigkeitsproben Plasmaproben umfassen und/oder wobei die Referenz-Körperflüssigkeitsproben Serumproben umfassen; und/oder
(d) die Referenz-Nichtkörperflüssigkeitsproben Zellproben umfassen und/oder wobei die Referenz-Nichtkörperflüssigkeitsproben Gewebeproben umfassen.

**13.** System, umfassend einen Controller, der computerlesbare Medien umfasst oder auf diese zugreifen kann, die nicht-flüchtige, computerausführbare Anweisungen umfassen, die, wenn sie von mindestens einem elektronischen Prozessor ausgeführt werden, zumindest Folgendes ausführen: das Verfahren nach einem der vorhergehenden Ansprüche.

**14.** System nach Anspruch 13, umfassend

(a) einen Nukleinsäuresequenzierer, der betriebsmäßig mit dem Controller verbunden ist, wobei der Nukleinsäuresequenzierer so konfiguriert ist, dass er Sequenzierungsablesungen bereitstellt, die von cfNA-Molekülen in den cfNA-Proben stammen; und/oder wobei der Nukleinsäuresequenzierer oder eine andere Systemkomponente so konfiguriert ist, dass er/sie Sequenzablesungen, die vom Nukleinsäuresequenzierer erzeugt wurden, in Familien von Sequenzablesungen gruppiert, wobei jede Familie Sequenzablesungen umfasst, die von einem jeweiligen cfNA-Molekül in den cfNA-Proben erzeugt wurden;
(b) eine Datenbank, die betriebsmäßig mit dem Controller verbunden ist, wobei die Datenbank eine oder mehrere Therapien umfasst, die hinsichtlich der Tumorursprung-Nukleinsäurevarianten indiziert sind;
(c) eine Probenvorbereitungskomponente, die betriebsmäßig mit dem Controller verbunden ist, wobei die Probenvorbereitungskomponente so konfiguriert ist, dass sie die cfNA-Moleküle in den cfNA-Proben vorbereitet, damit sie vom Nukleinsäuresequenzierer sequenziert werden können;
(d) eine Nukleinsäureamplifikationskomponente, die betriebsmäßig mit dem Controller verbunden ist, wobei die Nukleinsäureamplifikationskomponente so konfiguriert ist, dass sie zumindest angezielte Segmente der cfNA-Moleküle in den cfNA-Proben amplifiziert; und/oder
(e) eine Materialtransferkomponente, die betriebsmäßig mit dem Controller verbunden ist, wobei die Materialtransferkomponente so konfiguriert ist, dass sie ein oder mehrere Materialien zwischen zumindest dem Nukleinsäuresequenzierer und der Probenvorbereitungskomponente überträgt.

**15.** Computerlesbares Medium, das nicht-flüchtige, computerausführbare Anweisungen umfasst, die, wenn sie von mindestens einem elektronischen Prozessor ausgeführt werden, zumindest Folgendes ausführen: das Verfahren nach einem der vorhergehenden Ansprüche 1-12.

**Revendications**

**1.** Procédé de différenciation de variants d'acide nucléique d'origine tumorale et non tumorale dans un échantillon d'acide nucléique acellulaire (cfNA) obtenu à partir d'un sujet de test à l'aide au moins en partie d'un ordinateur, le procédé comprenant :

la génération ou la fourniture, par l'ordinateur, d'au moins un ensemble de données de variant tumoral comprenant une population de variants génétiques associés à une tumeur de référence, dans lequel l'ensemble de données de variant tumoral comprend la fréquence de données d'observation parmi des échantillons de référence qui comprennent des échantillons de liquide corporel de référence et des échantillons de liquide non corporel de référence pour un ou plusieurs variants génétiques associés à une tumeur de la population de variants génétiques associés à une tumeur de référence et dans lequel les échantillons de référence sont obtenus à partir d'un seul sujet de référence et/ou à partir de différents sujets de référence ayant un type de cancer identique ;
la détermination, par l'ordinateur, d'un ou plusieurs rapports de la fréquence de données d'observation entre les échantillons de référence pour un ou plusieurs variants génétiques associés à une tumeur de la population de variants génétiques associés à une tumeur de référence pour produire au moins un ensemble de données de variance et/ou de prévalence relative de fraction d'allèle mutant (MAF), dans lequel l'uniformité de la prévalence sur tous les types de cancer, la variation longitudinale des MAF au cours du temps et/ou la proportion dans des cancers hématologiques sont utilisées conjointement avec les rapports de la fréquence de données d'observation ;

la génération, par l'ordinateur, d'au moins un ensemble de probabilités d'origine non tumorale à partir de l'ensemble de données de variance et/ou de prévalence relative de MAF ; et,

l'utilisation de l'ensemble de probabilités d'origine non tumorale pour différencier des variants d'acide nucléique détectés dans l'échantillon de cfNA obtenu à partir du sujet de test comme étant des variants d'acide nucléique d'origine tumorale ou des variants d'acide nucléique d'origine non tumorale.

2. Procédé selon la revendication 1, dans lequel l'ensemble de données de prévalence relative est produit sur la base de

(a) la détermination, par l'ordinateur, de la prévalence relative d'un ou plusieurs variants génétiques associés à une tumeur observés dans un ou plusieurs échantillons de liquide corporel de référence par comparaison avec un ou plusieurs échantillons de liquide non corporel de référence ; ou

(b) la détermination, par l'ordinateur, d'une variation d'une valeur de fraction d'allèle mutant (MAF) et/ou d'au moins une statistique associée à celle-ci, pour au moins deux points dans le temps différents pour chacun des un ou plusieurs variants génétiques associés à une tumeur et/ou des variants génétiques non associés à une tumeur.

3. Procédé selon la revendication 1 ou la revendication 2, dans lequel l'au moins un ensemble de probabilités d'origine non tumorale est généré à partir de l'ensemble de données de prévalence relative et dans lequel la différenciation des variants d'acide nucléique d'origine tumorale et non tumorale dans l'échantillon de cfNA obtenu à partir du sujet de test comprend :

la classification, par l'ordinateur, d'au moins un premier variant d'acide nucléique détecté dans l'échantillon de cfNA obtenu à partir du sujet de test comme étant un variant d'acide nucléique d'origine tumorale lorsqu'une prévalence du premier variant d'acide nucléique détecté dans l'échantillon de cfNA est inférieure à un seuil de probabilité provenant de l'ensemble de probabilités d'origine non tumorale et

la classification, par l'ordinateur, d'au moins un second variant d'acide nucléique détecté dans l'échantillon de cfNA obtenu à partir du sujet de test comme étant un variant d'acide nucléique d'origine non tumorale lorsqu'une prévalence du second variant d'acide nucléique détecté dans l'échantillon de cfNA est supérieure à un seuil de probabilité provenant de l'ensemble de probabilités d'origine non tumorale.

4. Procédé selon une quelconque revendication précédente,

(a) comprenant l'identification de variants génétiques présents dans l'échantillon de cfNA à partir de lectures de séquençage provenant de molécules de cfNA de l'échantillon de cfNA, par exemple dans lequel les lectures de séquençage sont obtenues à partir de segments ciblés des molécules de cfNA de l'échantillon de cfNA ;

(b) dans lequel la population de variants génétiques associés à une tumeur de référence sont obtenus à partir des échantillons de référence ; et/ou

(c) dans lequel l'ensemble de données de variant tumoral comprend la fréquence de données d'observation parmi des échantillons de référence d'un type de cancer donné pour un ou plusieurs variants génétiques associés à une tumeur de la population de variants génétiques associés à une tumeur de référence.

5. Procédé selon une quelconque revendication précédente, comprenant l'entraînement d'un modèle d'apprentissage automatique à l'aide d'au moins une partie de la population de variants génétiques associés à une tumeur pour produire un modèle d'apprentissage automatique entraîné, dans lequel les variants d'acide nucléique d'origine tumorale et les variants d'acide nucléique d'origine non tumorale détectés dans l'échantillon de cfNA obtenu à partir du sujet de test sont différenciés les uns des autres à l'aide du modèle d'apprentissage automatique entraîné, éventuellement :

(a) dans lequel le modèle d'apprentissage automatique est entraîné à l'aide d'un ou plusieurs de : une régression logistique, une régression des probits, des arbres de décision, des forêts aléatoires, un boosting de gradient, un séparateur à vaste marge, les k plus proches voisins et un réseau neuronal ;

(b) comprenant le partage aléatoire de l'ensemble de données de variant tumoral en un ensemble de données d'entraînement et un ensemble de données de test ; et/ou

(c) dans lequel l'ensemble de données d'entraînement comprend environ 80 % de l'ensemble de données de variant tumoral et l'ensemble de données de test comprend environ 20 % de l'ensemble de données de variant tumoral.

6. Procédé selon une quelconque revendication précédente,

(a) comprenant l'utilisation d'un seuil de probabilité d'au moins environ un 30e centile pour un variant génétique donné en tant que seuil de coupure pour la classification ;
(b) comprenant de la mise en œuvre d'une régression logistique sur au moins l'un des rapports pour obtenir une probabilité donnée d'origine non tumorale ;
(c) dans lequel l'ensemble de données de variant tumoral comprend des données de fraction d'allèle mutant observées parmi des échantillons de référence pour un ou plusieurs variants génétiques associés à une tumeur de la population de variants génétiques associés à une tumeur de référence ; et/ou
(d) comprenant la normalisation de l'ensemble de données de variant tumoral à l'aide d'une ou plusieurs techniques de normalisation de données, par exemple dans lequel les techniques de normalisation de données comprennent une normalisation min-max et/ou une normalisation z-score.

7. Procédé selon une quelconque revendication précédente, dans lequel les échantillons de liquide non corporel de référence comprennent des échantillons de tissu tumoral de référence et/ou des échantillons de globules blancs de référence ; éventuellement dans lequel :

(a) un rapport de la fréquence de données d'observation d'un variant génétique donné dans les échantillons de liquide corporel de référence par rapport à la fréquence de données d'observation du variant génétique donné dans les échantillons de liquide non corporel de référence qui est supérieur à un (1,0) indique que le variant génétique donné est probablement un variant d'acide nucléique d'origine non tumorale, dans lequel les échantillons de liquide non corporel de référence comprennent des échantillons de tissu tumoral de référence ; ou
(b) un rapport de la fréquence de données d'observation d'un variant génétique donné dans les échantillons de liquide corporel de référence par rapport à la fréquence de données d'observation du variant génétique donné dans les échantillons de liquide non corporel de référence qui est inférieur à un (1,0) indique que le variant génétique donné est probablement un variant d'acide nucléique d'origine non tumorale, dans lequel les échantillons de liquide non corporel de référence comprennent des échantillons de globules blancs de référence.

8. Procédé selon une quelconque revendication précédente, dans lequel l'ensemble de probabilités d'origine non tumorale comprennent au moins un ensemble de probabilités d'origine hématopoïétique clonale.

9. Procédé selon une quelconque revendication précédente, comprenant la sélection d'une ou plusieurs thérapies pour traiter un type de cancer lorsqu'un ou plusieurs variants d'acide nucléique d'origine tumorale associés au type de cancer sont détectés dans l'échantillon de cfNA obtenu à partir du sujet de test.

10. Procédé selon une quelconque revendication précédente, dans lequel le type de cancer est choisi dans le groupe constitué par : un cancer du tractus biliaire, un cancer de la vessie, un carcinome à cellules transitionnelles, un carcinome urothélial, un cancer du cerveau, des gliomes, des astrocytomes, un carcinome du sein, un carcinome métaplasique, un cancer du col de l'utérus, un carcinome à cellules squameuses du col de l'utérus, un cancer rectal, un carcinome colorectal, un cancer du côlon, un cancer colorectal héréditaire sans polypose, des adénocarcinomes colorectaux, des tumeurs stromales gastrointestinales (GIST), un carcinome de l'endomètre, des sarcomes stromaux de l'endomètre,, un cancer de l'œsophage, un carcinome à cellules squameuses de l'œsophage, un adénocarcinome de l'œsophage, un mélanome oculaire, un mélanome uvéal, des carcinomes de la vésicule biliaire, des adénocarcinomes de la vésicule biliaire, un carcinome à cellules rénales, un carcinome à cellules rénales à cellules claires, un carcinome à cellules transitionnelles, des carcinomes urothéliaux, une tumeur de Wilms, une leucémie, une leucémie lymphoïde aiguë (LLA), une leucémie myéloïde aiguë (LMA), une leucémie lymphoïde chronique (LLC), une leucémie myéloïde chronique (LMC), une leucémie myélomonocytaire chronique (LMMC), un cancer du foie, un carcinome du foie, un hépatome, carcinome hépatocellulaire, un cholangiocarcinome, un hépatoblastome, un cancer du poumon, un cancer du poumon non à petites cellules (CPNPC), un mésothéliome, des lymphomes B, un lymphome non hodgkinien, un lymphome diffus à grandes cellules B, un lymphome à cellules du manteau, des lymphomes T, un lymphome non hodgkinien, un lymphome/leucémie lymphoblastique à précurseurs T, des lymphomes T périphériques, un myélome multiple, un carcinome du nasopharynx (CNP), un neuroblastome, un cancer de l'oropharynx, des carcinomes à cellules squameuses de la cavité orale, un ostéosarcome, un carcinome de l'ovaire, un cancer du pancréas, un adénocarcinome du canal pancréatique, des néoplasmes pseudopapillaires, des carcinomes à cellules acineuses, un cancer de la prostate, un adénocarcinome de la prostate, un cancer de la peau, un mélanome, un mélanome malin, un mélanome cutané, des carcinomes de l'intestin grêle, un cancer de l'estomac, un carcinome gastrique, une tumeur stromale gastrointestinale (GIST), un cancer de l'utérus et un sarcome de l'utérus.

11. Procédé selon une quelconque revendication précédente, dans lequel

(a) les variants génétiques associés à une tumeur de référence sont choisis dans le groupe constitué par : des variants mononucléotidiques (SNV), des insertions ou délétions (indels), des variants du nombre de copies (CNV), des fusions, des transversions, des translocations, des déphasages, des duplications, des expansions de répétitions et des variants épigénétiques ; et/ou

(b) les échantillons de référence comprennent au moins environ 25, au moins environ 50, au moins environ 100, au moins environ 200, au moins environ 300, au moins environ 400, au moins environ 500, au moins environ 600, au moins environ 700, au moins environ 800, au moins environ 900, au moins environ 1 000, au moins environ 5 000, au moins environ 10 000, au moins environ 15 000, au moins environ 20 000, au moins environ 25 000, au moins environ 30 000 ou plus d'échantillons de liquide corporel et/ou de liquide non corporel.

12. Procédé selon une quelconque revendication précédente, dans lequel

(a) l'échantillon de cfNA comprend de l'acide désoxyribonucléique acellulaire (cfDNA) et/ou dans lequel l'échantillon de cfNA comprend de l'acide ribonucléique cellulaire (cfRNA) ;

(b) le sujet de test est un sujet mammifère, par exemple dans lequel le sujet de test est un sujet humain ;

(c) les échantillons de liquide corporel de référence comprennent des échantillons de plasma et/ou dans lequel les échantillons de liquide corporel de référence comprennent des échantillons de sérum ; et/ou

(d) les échantillons de liquide corporel de référence comprennent des échantillons de cellules et/ou dans lequel les échantillons de liquide non corporel de référence comprennent des échantillons de tissu.

13. Système, comprenant un dispositif de commande comprenant, ou capable d'accéder à celui-ci, un support lisible par ordinateur comprenant des instructions exécutables par ordinateur non transitoires qui, lorsqu'elles sont exécutées par au moins un processeur électronique, effectuent au moins : le procédé selon l'une quelconque des revendications précédentes.

14. Système selon la revendication 13, comprenant

(a) un séquenceur d'acide nucléique connecté de manière fonctionnelle au dispositif de commande, lequel séquenceur d'acide nucléique est conçu pour fournir des lectures de séquençage provenant de molécules de cfNA dans les échantillons de cfNA ; et/ou dans lequel le séquenceur d'acide nucléique ou un autre composant du système est conçu pour grouper des lectures de séquence générées par le séquenceur d'acide nucléique en familles de lectures de séquence, chaque famille comprenant des lectures de séquence générées à partir d'une molécule de cfNA donnée dans les échantillons de cfNA ;

(b) une base de données connectée de manière fonctionnelle au dispositif de commande, laquelle base de données comprend une ou plusieurs thérapies indexées au variant d'acide nucléique d'origine tumorale ;

(c) un composant de préparation d'échantillon connecté de manière fonctionnelle au dispositif de commande, lequel composant de préparation d'échantillon est conçu pour préparer les molécules de cfNA dans les échantillons de cfNA à séquencer par le séquenceur d'acide nucléique ;

(d) un composant d'amplification d'acide nucléique connecté de manière fonctionnelle au dispositif de commande, lequel composant d'amplification d'acide nucléique est conçu pour amplifier au moins des segments ciblés des molécules de cfNA des échantillons de cfNA ; et/ou

(e) un composant de transfert de matière connecté de manière fonctionnelle au dispositif de commande, lequel composant de transfert de matière est conçu pour transférer une ou plusieurs matières entre le séquenceur d'acide nucléique et le composant de préparation d'échantillon.

15. Support lisible par ordinateur comprenant des instructions exécutables par ordinateur non transitoires qui, lorsqu'elles sont exécutées par au moins un processeur électronique, effectuent au moins : le procédé selon l'une quelconque des revendications 1-12.

## FIG. 1

100

102

┌─────────────────────────────────────────────────────────┐
│ DETERMINE RELATIVE PREVALENCE OF TUMOR-RELATED GENETIC │
│ VARIANTS │
└─────────────────────────────────────────────────────────┘

104

┌─────────────────────────────────────────────────────────┐
│ GENERATE A SET OF PROBABILITIES OF NON-TUMOR ORIGIN FROM THE │
│ RELATIVE PREVALENCE DATASET │
└─────────────────────────────────────────────────────────┘

106

┌─────────────────────────────────────────────────────────┐
│ USE THE SET OF PROBABILITIES OF NON-TUMOR ORIGIN TO │
│ DIFFERENTIATE NUCLEIC ACID VARIANTS AS BEING TUMOR ORIGIN OR │
│ NON-TUMOR ORIGIN │
└─────────────────────────────────────────────────────────┘

FIG. 2

200

202

GENERATE A TUMOR VARIANT DATASET THAT INCLUDES A POPULATION OF REFERENCE TUMOR-RELATED GENETIC VARIANTS AND FREQUENCY OF OBSERVANCE DATA AMONG REFERENCE BODILY FLUID SAMPLES AND REFERENCE NON-BODILY FLUID SAMPLES

204

DETERMINE RATIOS OF THE FREQUENCY OF OBSERVANCE DATA TO PRODUCE AT LEAST ONE MAF VARIANCE AND/OR RELATIVE PREVALENCE DATASET

206

GENERATE A SET OF PROBABILITIES OF NON-TUMOR ORIGIN FROM THE MAF VARIANCE AND/OR RELATIVE PREVALENCE DATASET

208

USE THE SET OF PROBABILITIES OF NON-TUMOR ORIGIN TO DIFFERENTIATE NUCLEIC ACID VARIANTS AS BEING TUMOR ORIGIN OR NON-TUMOR ORIGIN

## FIG. 3

300

302

RAW DATA

| PLASMA DATASETS | TISSUE DATASETS |

FEATURE ENGINEERING

| UNIFORMITY OF PREVALENCE ACROSS CANCER TYPES 306 | PLASMA VS TISSUE ENRICHMENT 304 | LONGITUDINAL MAF OVER TIME 303 | HEMATOLOGICAL MALIGNANCY PROPORTION 305 |

308

DATA NORMALIZATION

CLEAN-UP, LOG/YEO-JOHNSON TRANSFORMATION

310

MACHINE LEARNING

LOGISTIC REGRESSION, RANDOM FOREST, DEEP LEARNING

312

CLASSIFICATION

CLASSIFICATION OF TUMOR AND NON-TUMOR VARIANT

FIG. 4

## FIG. 5

```
┌─────────────────────┐        ┌─────────────────────┐        ┌─────────────────────┐
│  UNCLASSIFIED DATA  │ ═════▷ │     ML MODULE       │ ═════▷ │ PREDICTION RESULT   │
│        610          │        │        430          │        │        620          │
└─────────────────────┘        └─────────────────────┘        └─────────────────────┘
```

FIG. 6

**FIG. 7**

700

SERVER 702

PROCESSOR 704

MEMORY 706

PROGRAM
PRODUCT 708

DATA FILES
710

724

NETWORK 712

716

722

720

718

714

**FIG. 8**

A **Std of MAFs between visits**

B

**Longitudinal MAFs**

ROC AUC: 0.90

C

Confusion Matrix

**FIG. 9**

# FIG. 10

A

**Heme Prevalence**

B

Confusion Matrix

ROC AUC: 0.94

EP 4 118 653 B1

FIG. 11

FIG. 12

EP 4 118 653 B1

A

**Tumor and Non-Tumor Variant Classifier**

B

**PPA with WBC (Non-Tumor)**
Sensitivity: 70% (30/43)  WBC + | CH pred +
Specificity: 63.8% (30/47) CH pred + | WBC +

**PPA with Plasma only (Tumor)**
Sensitivity 84.8% (95/112) Tumor Pred + | Plasma Only
Specificity: 88.0% (95/108) Plasma Only | Tumor Pred +

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019236478 A1
- US 20010053519 A
- US 20110160078 A
- US 6582908 B
- US 7537898 B
- US 9598731 B
- US 2019042882 W

### Non-patent literature cited in the description

- **SUKHAI et al.** *J Mol Diagn.,* 2019, vol. 21 (2), 261-273
- **RAZAVI et al.** High-intensity sequencing reveals the sources of plasma circulating cell-free DNA variants. *Nature Medicine,* 2019, vol. 25, 1928-1937
- **COOMBS et al.** Therapy-related clonal hematopoiesis in patients with non-hematologic cancers is common and impacts clinical outcome. *Cell Stem Cell,* 2017, vol. 21 (3), 374-382
- **HUBBELL et al.** Cell-free DNA (cfDNA) fragment length patterns of tumor- and blood-derived variants in participants with and without cancer. *2019 AACR Meeting, Abstract 3372,* 29 March 2019
- **LI et al.** Ultra-deep next generation sequencing of plasma cell-free DNA in patients with advanced lung cancers: results from the Actionable Genome Consortium. *Ann. Oncol.,* 2019, vol. 30 (4), 597-603
- **PETERSON.** Computer Networks: A Systems Approach. Morgan Kaufmann, 2011
- **KUROSE.** Computer Networking: A Top-Down Approach. Pearson, 2016
- **ELMASRI.** Fundamentals of Database Systems. Addison Wesley, 2010
- **CORONEL.** Database Systems: Design, Implementation, & Management. Cengage Learning, 2014
- **TUCKER.** Programming Languages. McGraw-Hill Science/Engineering/Math, 2006
- **RHOTON.** Cloud Computing Architected: Solution Design Handbook. Recursive Press, 2011
- **JAISWAL et al.** Age-related clonal hematopoiesis associated with adverse outcomes. *N Engl J Med.,* 25 December 2014, vol. 371 (26), 2488-98
- **ZEHIR et al.** Mutational landscape of metastatic cancer revealed from prospective clinical sequencing of 10,000 patients. *Nat Med.,* June 2017, vol. 23 (6), 703-713
- **TATE et al.** COSMIC: the Catalogue Of Somatic Mutations In Cancer. *Nucleic Acids Res.,* 08 January 2019, vol. 47 (D1), D941-D947
- **YEN et al.** Analysis of clonal hematopoiesis in cell-free DNA of advanced cancer patients. *Poster #5396, AACR,* 2019